(19) **Europäisches Patentamt**
**European Patent Office**
**Office européen des brevets**

(11) **EP 2 369 338 B1**

(12) **EUROPEAN PATENT SPECIFICATION**

(45) Date of publication and mention
of the grant of the patent:
**02.11.2016  Bulletin 2016/44**

(51) Int Cl.:
*G01N 33/22* (2006.01)    *C10L 3/06* (2006.01)
*C10L 1/00* (2006.01)

(21) Application number: **11159687.0**

(22) Date of filing: **24.03.2011**

(54) **Calorific value calculation formula creation system, method of creating calorific value calculation formula, calorific value measurement system and method of measuring calorific value**

Formelerzeugungssystem zur Kalorienberechnung, Verfahren zur Erzeugung der Formel zur Kalorienberechnung, Messsystem des Kalorienwerts und Verfahren zum Messen des Kalorienwerts

Système de création de formule de calcul de la valeur calorifique, procédé de création de formule de calcul de la valeur calorifique, système de mesure de la valeur calorifique et procédé de mesure de la valeur calorifique

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **26.03.2010  JP 2010073439**

(43) Date of publication of application:
**28.09.2011  Bulletin 2011/39**

(73) Proprietor: **Azbil Corporation**
**Chiyoda-ku**
**Tokyo 100-6419 (JP)**

(72) Inventors:
• **Mutou, Hiroyuki**
**Tokyo 100-6419 (JP)**
• **Ooishi, Yasuharu**
**Tokyo 100-6419 (JP)**
• **Seita, Misako**
**Tokyo 100-6419 (JP)**

(74) Representative: **Lees, Gregory Alexander et al**
**Dehns**
**St Bride's House**
**10 Salisbury Square**
**London EC4Y 8JD (GB)**

(56) References cited:
**EP-A1- 1 947 450**

• **A.J. SMOLA, B. SCHÖLKOPF: "A Tutorial on Support Vector Regression", NEUROCOLT TECHNICAL REPORT (NC-TR-98-030), 1998, XP002638775,**
• **UDINA S ET AL: "A micromachined thermoelectric sensor for natural gas analysis: Thermal model and experimental results", SENSORS AND ACTUATORS B, ELSEVIER SEQUOIA S.A., LAUSANNE, CH, vol. 134, no. 2, 25 September 2008 (2008-09-25), pages 551-558, XP025429938, ISSN: 0925-4005, DOI: DOI:10.1016/J.SNB.2008.05.043 [retrieved on 2008-06-08]**
• **LOUBAR ET AL: "A combustionless determination method for combustion properties of natural gases", FUEL, IPC SCIENCE AND TECHNOLOGY PRESS, GUILDFORD, GB, vol. 86, no. 16, 9 October 2007 (2007-10-09), pages 2535-2544, XP022293299, ISSN: 0016-2361, DOI: DOI:10.1016/J.FUEL.2007.02.024**
• **PETER ULBIG, DETLEV HOBURG: "DETERMINATION OF THE CALORIFIC VALUE OF NATURAL GAS BY DIFFERENT METHODS", THERMOCHIMICA ACTA, vol. 382, 2002, pages 27-35, XP002639490,**

**Description**

Technical Field

The present invention relates to a gas examination technique, and more particularly, to a calorific value calculation formula creation system, a method of creating a calorific value calculation formula, a calorific value measurement system, and a method of measuring a calorific value.

Related Art

In the related art, when the calorific value of a mixed gas is calculated, it is necessary to analyze the components of the mixed gas using, for example, an expensive gas chromatography device.
In addition, a method has been proposed which measures the thermal conductivity of a mixed gas and the speed of sound in the mixed gas to calculate the percentage of components, such as methane ($CH_4$), propane ($C_3H_8$), nitrogen ($N_2$), and carbon dioxide ($CO_2$), included in the mixed gas, thereby calculating the calorific value of the mixed gas (for example, see JP-T-2004-514138). However, in the method disclosed in JP-T-2004-514138, an expensive sound speed sensor for measuring the speed of sound is required in addition to a sensor for measuring thermal conductivity.
There is also proposed a method of determining the calorific value of a mixed gas based on the thermal conductivity of the mixed gas (for example, see EP 1947540).

SUMMARY

Viewed from a first aspect, the present invention provides a resistance measurement system comprising:

a resistance calculation formula storage device which is arranged to store a resistance calculation formula, wherein the resistance calculation formula is represented by:

a first temperature detected by a temperature measurement element coming into contact with a calibration gas, the first temperature being set as an independent variable in the resistance calculation formula; and
a first resistance of a heater element coming into contact with the calibration gas, the resistance being set as a dependent variable in the resistance calculation formula,

a first measurement module which is arranged to obtain a measured value of the first temperature and a measured value of the first resistance;
a correction coefficient calculation module which is arranged to:

substitute the measured value of the first temperature into the first temperature that is set as the independent variable in the resistance calculation formula so as to obtain a calculated value of the first resistance; and
calculate a correction coefficient that represents a ratio between the measured value of the first resistance and the calculated value of the first resistance; and

a measurement correction module which corrects a measured value of the first resistance, based on the correction coefficient.

Viewed from a second aspect, the present invention provides a method of measuring a resistance, the method comprising:

(a) preparing a resistance calculation formula, wherein the resistance calculation formula is represented by:

a first temperature detected by a temperature measurement element coming into contact with a calibration gas, the first temperature being set as an independent variable in the resistance calculation formula; and
a first resistance of a heater element coming into contact with the calibration gas, the resistance being set as a dependent variable in the resistance calculation formula,

(b) obtaining a measured value of the first temperature and a measured value of the first resistance;
(c) substituting the measured value of the first temperature into the first temperature that is set as the independent variable in the resistance calculation formula so as to obtain a calculated value of the first resistance; and
(d) calculating a correction coefficient that represents a ratio between the measured value of the first resistance and the calculated value of the first resistance; and

(e) correcting a measured value of the first resistance, based on the correction coefficient.

The present invention also provides a calorific value calculation formula creation system, a method of creating a calorific value calculation formula, a calorific value measurement system, and a method of measuring a calorific value, which include the resistance measurement system or method of the first and second aspects.

BRIEF DESCRIPTION OF THE DRAWINGS

Fig. 1 is a perspective view illustrating a microchip according to a first embodiment of the invention;
Fig. 2 is a cross-sectional view illustrating the microchip according to the first embodiment of the invention taken along the line II-II of Fig. 1;
Fig. 3 is a circuit diagram illustrating a heater element according to the first embodiment of the invention;
Fig. 4 is a circuit diagram a heat-retaining element according to the first embodiment of the invention;
Fig. 5 is a graph illustrating the relationship between the radiation coefficient of gas and the temperature of the heater element according to the first embodiment of the invention;
Fig. 6 is a first schematic diagram of a calorific value calculation formula creation system according to the first embodiment of the invention;
Fig. 7 is a second schematic diagram of the calorific value calculation formula creation system according to the first embodiment of the invention;
Fig. 8 is a flowchart illustrating a method of creating a calorific value calculation formula according to the first embodiment of the invention;
Fig. 9 is a schematic diagram of a calorific value calculation formula creation system according to a second embodiment of the invention;
Fig. 10 is a schematic diagram of a calorific value calculation formula creation system according to a third embodiment of the invention;
Fig. 11 is a schematic diagram illustrating a calorific value measurement system according to a fourth embodiment of the invention;
Fig. 12 is a flowchart illustrating a method of measuring a calorific value according to the fourth embodiment of the invention;
Fig. 13 is a schematic diagram illustrating a calorific value measurement system according to a fifth embodiment of the invention;
Fig. 14 is a schematic diagram illustrating a calorific value measurement system according to a sixth embodiment of the invention; and
Fig. 15 is a graph illustrating an error from the true value of the calculated calorific value of a sample mixed gas according to Example 2 of the embodiment.

DETAILED DESCRIPTION OF EXEMPLARY EMBODIMENTS

Hereinafter, exemplary embodiments of the invention will be described. In the following description of the drawings, the same or similar components are denoted by the same or similar reference numerals. However, the drawings are schematically illustrated. Therefore, for example, detailed dimensions need to be determined in the light of the following description. In addition, in the drawings, the same components may have different dimensions or ratios.

(First embodiment)

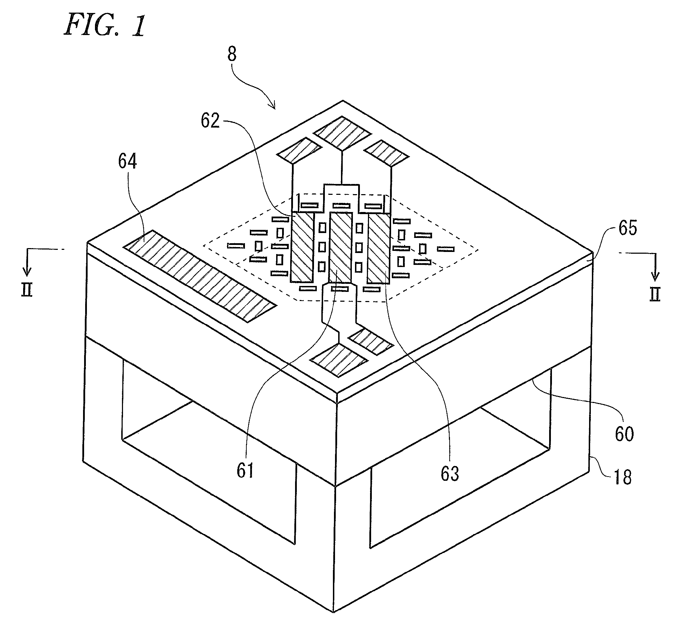

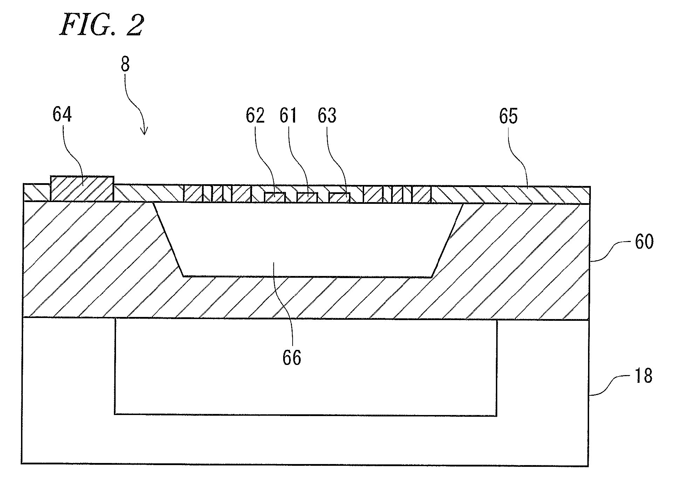

First, a microchip 8 used in a gas property value measurement system according to a first embodiment will be described with reference to Fig. 1, which is a perspective view, and Fig. 2, which is a cross-sectional view taken along the line II-II. The microchip 8 includes a substrate 60 having a cavity 66 provided therein and an insulating film 65 that is provided on the substrate 60 so as to cover the cavity 66. The thickness of the substrate 60 is, for example, 0.5 mm. In addition, the substrate 60 has, for example, a size of about 1.5 mm x 1.5 mm. A portion of the insulating film 65 covering the cavity 66 is a heat insulating diaphragm. The microchip 8 further includes a heater element 61 that is provided in the diaphragm portion of the insulating film 65, first and second temperature measurement elements 62 and 63 that are provided in the diaphragm portion of the insulating film 65 with the heater element 61 interposed therebetween, and a heat-retaining element 64 that is provided on the substrate 60.

The heater element 61 is arranged at the center of the diaphragm portion of the insulating film 65 covering the cavity 66. The heater element 61 is, for example, a resistor and is supplied with power to generate heat, thereby heating an atmospheric gas coming into contact with the heater element 61. The first temperature measurement element 62 and the second temperature measurement element 63 are, for example, electronic elements such as a passive element of a resistor or the like and are supplied with a weak voltage not enough to generate self-heating to output an electric signal depending on the gas temperature of an atmospheric gas. Not generating self-heating means that the temperatures of the first temperature measurement element 62 and the second temperature measurement element 63 are close to the atmospheric temperature. Hereinafter, an example will be described in which an output signal of the first temperature measurement element 62 is used, but the invention is not limited thereto. For example, an average value of an output signal of the first temperature measurement element 62 and an output signal of the second temperature measurement element 63 may be used as the output signal of the temperature measurement element.

The heat-retaining element 64 is, for example, a resistor and is supplied with power to generate heat, thereby constantly maintaining the temperature of the substrate 60 to, for example, 60°C. As a material for the substrate 60, silicon (Si) or the like can be used. As a material for the insulating film 65, silicon oxide ($SiO_2$) or the like can be used. The cavity 66 is formed by anisotropic etching or the like. In addition, platinum (Pt) or the like can be used as the materials for the heater element 61, the first temperature measurement element 62, the second temperature measurement element 63, and the heat-retaining element 64, and these can be formed using a lithography method. In addition, the heater element 61, the first temperature measurement element 62, and the second temperature measurement element 63 may be formed of the same members.

The microchip 8 is fixed to a container, such as a chamber filled up with an atmosphere gas, with a heat insulating member 18 which is provided on the bottom of the microchip 8 interposed therebetween. When the microchip 8 is fixed to, for example, the chamber with the heat insulating member 18 interposed therebetween, the temperature of the microchip 8 is less likely to be affected by a variation in the temperature of the inner wall of the chamber. For example, the thermal conductivity of the heat insulating member 18 made of glass is equal to or less than 1.0 W/(m·K).

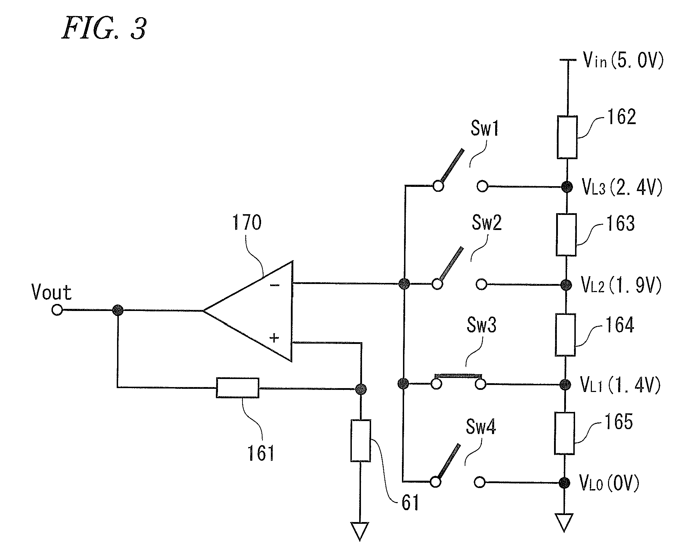

As shown in Fig. 3, the heater element 61 has one end that is electrically connected to, for example, a positive (+) input terminal of an operational amplifier 170 and the other end that is connected to the ground. A resistive element 161 is connected in parallel to the positive input terminal and an output terminal of the operational amplifier 170. A negative (-) input terminal of the operational amplifier 170 is electrically connected between a resistive element 162 and a resistive element 163 connected in series to each other, between the resistive element 163 and a resistive element 164 connected in series to each other, between the resistive element 164 and a resistive element 165 connected in series to each other, or to an earth terminal of the resistive element 165. The resistance values of the resistive elements 162 to 165 are appropriately determined, thereby for example, when a voltage Vin of 5.0 V is applied to one end of the resistive element 162, a voltage $V_{L3}$ of, for example, 2.4 V is generated between the resistive element 163 and the resistive element 162. In addition, a voltage $V_{L2}$ of, for example, 1.9 V is generated between the resistive element 164 and the resistive element 163 and a voltage $V_{L1}$ of, for example, 1.4 V is generated between the resistive element 165 and the resistive element 164.

A switch SW1 is provided between the positive input terminal of the operational amplifier and a point between the resistive element 162 and the resistive element 163, and a switch SW2 is provided between the positive input terminal of the operational amplifier and a point between the resistive element 163 and the resistive element 164. In addition, a switch SW3 is provided between the positive input terminal of the operational amplifier and a point between the resistive element 164 and the resistive element 165, and a switch SW4 is provided between the ground terminal of the resistive element 165 and the positive input terminal of the operational amplifier.

When a voltage $V_{L3}$ of 2.4 V is applied to the negative input terminal of the operational amplifier 170, only the switch SW1 is energized and the switches SW2, SW3, and SW4 are disconnected. When a voltage $V_{L2}$ of 1.9 V is applied to the negative input terminal of the operational amplifier 170, only the switch SW2 is energized and the switches SW1, SW3, and SW4 are disconnected. When a voltage $V_{L1}$ of 1.4 V is applied to the negative input terminal of the operational amplifier 170, only the switch SW3 is energized and the switches SW1, SW2, and SW4 are disconnected. When a voltage $V_{L0}$ of 0 V is applied to the negative input terminal of the operational amplifier 170, only the switch SW4 is energized and the switches SW1, SW2, and SW3 are disconnected. Accordingly, any of 0 V and three stages of voltages can be applied to the negative input terminal of the operational amplifier 170 by turning on and off of the switches SW1, SW2, SW3, and SW4. Therefore, an application voltage which decides the heat generating temperature of the heater element 61 can be set in three stages by turning on and off of the switches SW1, SW2, SW3, and SW4.

Here, the temperature of the heater element 61 when a voltage $V_{L1}$ of 1.4 V is applied to the negative input terminal of the operational amplifier 170 is denoted by $T_{H1}$. The temperature of the heater element 61 when a voltage $V_{L2}$ of 1.9 V is applied to the negative input terminal of the operational amplifier 170 is denoted by $T_{H2}$. The temperature of the heater element 61 when a voltage $V_{L3}$ of 2.4 V is applied to the negative input terminal of the operational amplifier 170 is denoted by $T_{H3}$.

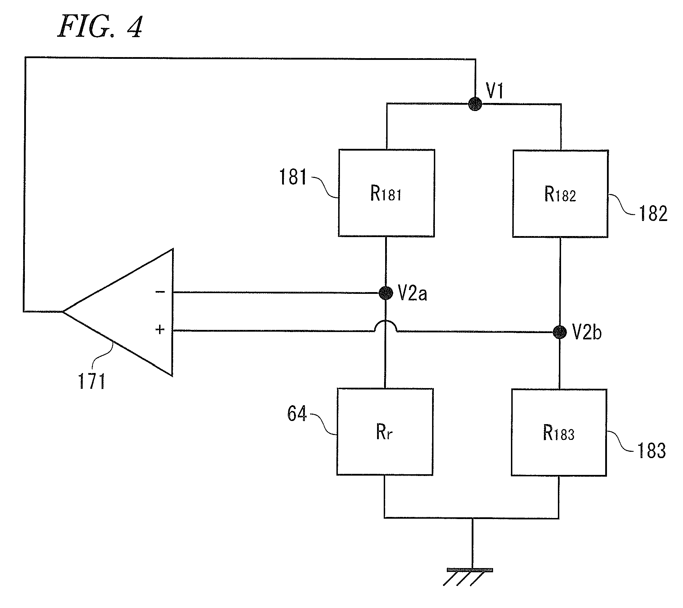

As shown in Fig. 4, the heat-retaining element 64 constitutes a portion of a resistance bridge circuit. The resistance bridge circuit includes: a resistive element 181 connected in series to the heat-retaining element 64; and resistive elements 182 and 183 connected in parallel to the heat-retaining element 64 and the resistive element 181. Here, the resistance value of the heat-retaining element 64 is denoted by Rr, and the fixed resistance values of the resistive elements 181, 182, and 183 are denoted by $R_{181}$, $R_{182}$, and $R_{183}$, respectively. An operational amplifier 171 is connected to the resistance bridge circuit. A bridge driving voltage $V_1$ is feedback-controlled so that a bridge voltage $V_{2a}$ between the resistive element 181 and the heat-retaining element 64 becomes equal to a bridge voltage $V_{2b}$ between the resistive element 182 and the resistive element 183. Accordingly, the resistance value Rr of the heat-retaining element 64 becomes constant and the heat-retaining element 64 generates heat at a certain temperature.

The resistance value of the heater element 61 shown in Figs. 1 and 2 varies depending on the temperature of the heater element 61. The relationship between the temperature $T_H$ of the heater element 61 and the resistance $R_H$ of the heater element 61 is represented by the following Expression (1):

$$R_H = R_{H\_STD} \times [1 + \alpha_H(T_H - T_{H\_STD}) + \beta_H(T_H - T_{H\_STD})^2] \qquad \ldots(1)$$

where the symbol $T_{H\_STD}$ represents the standard temperature of the heater element 61 and is, for example, 20°C. The symbol $R_{H\_STD}$ represents the resistance value of the heater element 61 which is measured at the standard temperature $T_{H\_STD}$ in advance. The symbol $\alpha_H$ represents a primary resistance-temperature coefficient. The symbol $\beta_H$ represents a secondary resistance-temperature coefficient.

The resistance value $R_H$ of the heater element 61 is calculated from the driving power $P_H$ of the heater element 61 and the on-current $I_H$ of the heater element 61 by the following Expression (2):

$$R_H = P_H/I_H^2. \qquad \ldots(2)$$

Alternatively, the resistance value $R_H$ of the heater element 61 is calculated from a voltage $V_H$ applied to the heater element 61 and the on-current $I_H$ of the heater element 61 by the following Expression (3):

$$R_H = V_H/I_H. \qquad \ldots(3)$$

Here, the temperature $T_H$ of the heater element 61 is stabilized when the heater element 61 and the atmospheric gas are thermally balanced. The thermally balanced state is a state in which the heat generation of the heater element 61 and the heat loss to the atmospheric gas from the heater element 61 are balanced. As shown in the following Expression (4), by dividing driving power $P_H$ of the heater element 61 in the balanced state by a difference $\Delta T_H$ between the temperature $T_H$ of the heater element 61 and the temperature $T_I$ of the atmospheric gas, a radiation coefficient $M_I$ of the atmospheric gas is obtained. The unit of the radiation coefficient $M_I$ is, for example, W/°C.

$$M_I = P_H/(T_H - T_r)$$

$$= P_H/\Delta T_H \qquad \ldots(4)$$

From the above Expression (1), the temperature $T_H$ of the heater element 61 is represented by the following Expression (5):

$$T_H = (1/2\beta_H) \times [-\alpha_H + [\alpha_H^2 - 4\beta_H(1 - R_H/R_{H\_STD})]^{1/2}] + T_{H\_STD} \qquad \ldots(5)$$

Accordingly, the difference $\Delta T_H$ between the temperature $T_H$ of the heater element 61 and the temperature $T_I$ of the atmospheric gas is represented by the following Expression (6):

$$\Delta T_H = (1/2\beta_H) \times [-\alpha_H + [\alpha_H^2 - 4\beta_H(1 - R_H/R_{H\_STD})]^{1/2}] + T_{H\_STD} - T_I \qquad \ldots(6)$$

The temperature $T_I$ of the atmospheric gas is close to the temperature $T_I$ of the first temperature measurement element 62 which is supplied with power enough not to generate self-heating. The relationship between the temperature $T_I$ of the first temperature measurement element 62 and the resistance $R_I$ of the first temperature measurement element 62 is represented by the following Expression (7):

$$R_I = R_{I\_STD} \times [1 + \alpha_I(T_I - T_{I\_STD}) + \beta_I(T_I - T_{I\_STD})^2] \qquad \ldots(7)$$

where the symbol $T_{I\_STD}$ represents the standard temperature of the first temperature measurement element 62 and is, for example, 20°C. The symbol $R_{I\_STD}$ represents the resistance value of the first temperature measurement element 62 which is measured at the standard temperature $T_{I\_STD}$ in advance. The symbol $\alpha_I$ represents a primary resistance-temperature coefficient. The symbol $\beta_I$ represents a secondary resistance-temperature coefficient. From the above Expression (7), the temperature $T_I$ of the first temperature measurement element 62 is represented by the following Expression (8):

$$T_I = (1/2\beta_I) \times [-\alpha_I + [\alpha_I^2 - 4\beta_I(1 - R_I/R_{I\_STD})]^{1/2}] + T_{I\_STD} \qquad \ldots(8)$$

Accordingly, the radiation coefficient $M_I$ of the atmospheric gas is represented by the following Expression (9):

$$M_I = P_H/\Delta T_H = P_H/[(1/2\beta_H)[-\alpha_H + [\alpha_H^2 - 4\beta_H(1 - R_H/R_{H\_STD})]^{1/2}] + T_{H\_STD} -$$

$$(1/2\beta_I)[-\alpha_I + [\alpha_I^2 - 4\beta_I(1 - R_I/R_{I\_STD})]^{1/2}] - T_{I\_STD}] \qquad \ldots(9)$$

Since the value of the on-current $I_H$ of the heater element 61 and the value of the driving power $P_H$ or the voltage $V_H$ can be measured, it is possible to calculate the value of the resistance $R_H$ of the heater element 61 from the above Expression (2) or (3). Similarly, it is possible to calculate the value of the resistance $R_I$ of the first temperature measurement element 62. Therefore, it is possible to calculate the radiation coefficient $M_I$ of the atmospheric gas from the above Expression (9) by using the microchip 8.

Since the heat-retaining element 64 maintains the temperature of the substrate 60 to be constant, the temperature of the atmosphere gas in the vicinity of the microchip 8 before the heater element 61 generates heat is approximate to the constant temperature of the substrate 60. Therefore, a variation in the temperature of the atmosphere gas before the heater element 61 generates heat is prevented. Since the heater element 61 heats the atmosphere gas whose temperature variation has been prevented, it is possible to calculate the radiation coefficient $M_I$ with high accuracy.

It is assumed that the atmosphere gas is a mixed gas and the mixed gas includes four kinds of gas components A, B, C, and D. The sum of the volume ratio $V_A$ of the gas A, the volume ratio $V_B$ of the gas B, the volume ratio $V_C$ of the gas C, and the volume ratio $V_D$ of the gas D is 1, as represented by the following Expression (10):

$$V_A + V_B + V_C + V_D = 1. \qquad \ldots(10)$$

When the calorific value of the gas A per unit volume is $K_A$, the calorific value of the gas B per unit volume is $K_B$, the calorific value of the gas C per unit volume is $K_C$, and the calorific value of the gas D per unit volume is $K_D$, the calorific value Q of the mixed gas per unit volume is the sum of the values obtained by multiplying the volume ratios of the gas components by the calorific values of the gas components per unit volume. Therefore, the calorific value Q of the mixed gas per unit volume is represented by the following Expression (11):

$$Q = K_A \times V_A + K_B \times V_B + K_C \times V_C + K_D \times V_D. \qquad \ldots(11)$$

The unit of the calorific value per unit volume is, for example, $MJ/m^3$.

When the radiation coefficient of the gas A is $M_A$, the radiation coefficient of the gas B is $M_B$, the radiation coefficient of the gas C is $M_C$, and the radiation coefficient of the gas D is $M_D$, the radiation coefficient $M_I$ of the mixed gas is the sum of the values obtained by multiplying the volume ratios of the gas components by the radiation coefficients of the gas components. Therefore, the radiation coefficient $M_I$ of the mixed gas is represented by the following Expression (12):

$$M_I = M_A \times V_A + M_B \times V_B + M_C \times V_C + M_D \times V_D. \qquad \ldots(12)$$

Further, since the radiation coefficient of gas depends on the heat generating temperature $T_H$ of the heater element 61, the radiation coefficient $M_I$ of the mixed gas is represented as a function of the temperature $T_H$ of the heater element 61 by the following Expression (13):

$$M_I(T_H)=M_A(T_H)\times V_A+M_B(T_H)\times V_B+M_C(T_H)\times V_C+M_D(T_H)\times V_D \qquad \ldots(13)$$

Accordingly, the radiation coefficient $M_I(T_{H1})$ of the mixed gas when the temperature of the heater element 61 is $T_{H1}$ is represented by the following Expression (14). The radiation coefficient $M_I(T_{H2})$ of the mixed gas when the temperature of the heater element 61 is $T_{H2}$ is represented by the following Expression (15), and the radiation coefficient $M_I(T_{H3})$ of the mixed gas when the temperature of the heater element 61 is $T_{H3}$ is represented by the following Expression (16).

$$M_I(T_{H1})=M_A(T_{H1})\times V_A+M_B(T_{H1})\times V_B+M_C(T_{H1})\times V_C+M_D(T_{H1})\times V_D \qquad \ldots(14)$$

$$M_I(T_{H2})=M_A(T_{H2})\times V_A+M_B(T_{H2})\times V_B+M_C(T_{H2})\times V_C+M_D(T_{H2})\times V_D \qquad \ldots(15)$$

$$M_I(T_{H3})=M_A(T_{H3})\times V_A+M_B(T_{H3})\times V_B+M_C(T_{H3})\times V_C+M_D(T_{H3})\times V_D \qquad \ldots(16)$$

Here, when the radiation coefficients $M_A(T_H)$, $M_B(T_H)$, $M_C(T_H)$, and $M_D(T_H)$ of the gas components relative to the temperature $T_H$ of the heater element 61 have nonlinearity, the above Expressions (14) to (16) have a linear independent relationship. In addition, even when the radiation coefficients $M_A(T_H)$, $M_B(T_H)$, $M_C(T_H)$, and $M_D(T_H)$ of the gas components relative to the temperature $T_H$ of the heater element 61 have linearity, when the change ratios of the radiation coefficients $M_A(T_H)$, $M_B(T_H)$, $M_C(T_H)$, and $M_D(T_H)$ of the gas components relative to the temperature $T_H$ of the heater element 61 are different from each other, the above Expressions (14) to (16) have a linear independent relationship. Further, when Expressions (14) to (16) have a linear independent relationship, Expressions (10) and (14) to (16) have a linear independent relationship.

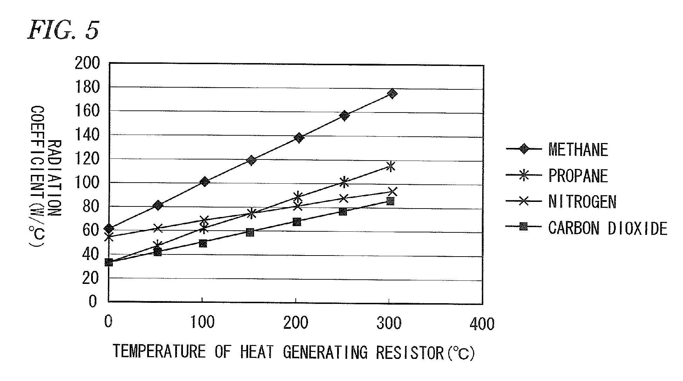

Fig. 5 is a graph illustrating the relationship between the radiation coefficients of methane ($CH_4$), propane ($C_3H_8$), nitrogen ($N_2$), and carbon dioxide ($CO_2$) which are contained in natural gas and the temperature of the heater element 61 which is a heat generating resistor. The radiation coefficients of the gas components of methane ($CH_4$), propane ($C_3H_8$), nitrogen ($N_2$), and carbon dioxide ($CO_2$) relative to the temperature of the heater element 61 have linearity. However, the change ratio of the radiation coefficient relative to the temperature of the heater element 61 is different for each of the cases of methane ($CH_4$), propane ($C_3H_8$), nitrogen ($N_2$), and carbon dioxide ($CO_2$). Therefore, when the gas components of the mixed gas are methane ($CH_4$), propane ($C_3H_8$), nitrogen ($N_2$), and carbon dioxide ($CO_2$), the above Expressions (14) to (16) have a linear independent relationship.

The values of the radiation coefficients $M_A(T_{H1})$, $M_B(T_{H1})$, $M_C(T_{H1})$, $M_D(T_{H1})$, $M_A(T_{H2})$, $M_B(T_{H2})$, $M_C(T_{H2})$, $M_D(T_{H2})$, $M_A(T_{H3})$, $M_B(T_{H3})$, $M_C(T_{H3})$, and $M_D(T_{H3})$ of the gas components in Expressions (14) to (16) can be obtained in advance by measurement or the like. Accordingly, when the simultaneous equations in Expressions (10) and (14) to (16) are solved, a volume ratio $V_A$ of a gas A, a volume ratio $V_B$ of a gas B, a volume ratio $V_C$ of a gas C, and a volume ratio $V_D$ of a gas D are respectively given as functions of the radiation coefficients $M_I(T_{H1})$, $M_I(T_{H2})$, and $M_I(T_{H3})$ of the mixed gas as shown in the following Expressions (17) to (20). In the following Expressions (17) to (20), the symbol n represents a natural number and the symbol $f_n$ represents a function.

$$V_A=f_1[M_I(T_{H1}), M_I(T_{H2}), M_I(T_{H3})] \qquad \ldots(17)$$

$$V_B=f_2[M_I(T_{H1}), M_I(T_{H2}), M_I(T_{H3})] \qquad \ldots(18)$$

$$V_C=f_3[M_I(T_{H1}), M_I(T_{H2}), M_I(T_{H3})] \qquad \ldots(19)$$

$$V_D=f_4[M_I(T_{H1}), M_I(T_{H2}), M_I(T_{H3})] \qquad \ldots(20)$$

Here, by substituting Expressions (17) to (20) into the above Expression (11), the following Expression (21) is obtained.

$$Q = K_A \times V_A + K_B \times V_B + K_C \times V_C + K_D \times V_D$$

$$= K_A \times f_1[M_I(T_{H1}), M_I(T_{H2}), M_I(T_{H3})] + K_B \times f_2[M_I(T_{H1}), M_I(T_{H2}), M_I(T_{H3})] +$$

$$K_C \times f_3[M_I(T_{H1}), M_I(T_{H2}), M_I(T_{H3})] + K_D \times f_4[M_I(T_{H1}), M_I(T_{H2}), M_I(T_{H3})]$$

$$\ldots(21)$$

As shown in the above Expression (21), the calorific value Q per unit volume of the mixed gas is represented by an equation which has as variables the radiation coefficients $M_I(T_{H1})$, $M_I(TH_2)$, and $M_I(T_{H3})$ of the mixed gas when the heat generating temperature of the heater element 61 is $T_{H1}$, $T_{H2}$, and $T_{H3}$. Therefore, the calorific value Q of the mixed gas is represented by the following Expression (22) where the symbol g represents a function.

$$Q = g[M_I(T_{H1}), M_I(T_{H2}), M_I(T_{H3})] \qquad \ldots(22)$$

Accordingly, the inventors have found out that, regarding the mixed gas formed of the gas A, the gas B, the gas C, and the gas D, when the above Expression (22) is achieved in advance, the calorific value Q per unit volume of an examination target mixed gas whose volume ratio $V_A$ of the gas A, the volume ratio $V_B$ of the gas B, the volume ratio $V_C$ of the gas C, and the volume ratio $V_D$ of the gas D are unknown can be easily calculated. In greater detail, the radiation coefficients $M_I(T_{H1})$, $M_I(T_{H2})$, and $M_I(T_{H3})$ of the examination target mixed gas when the heat generating temperature of the heater element 61 is $T_{H1}$, $T_{H2}$, and $T_{H3}$ are measured and substituted into Expression (22) to uniquely obtain the calorific value Q of the examination target mixed gas.

In addition, the radiation coefficient $M_I$ of the mixed gas depends on the resistance $R_H$ of the heater element 61 and the resistance $R_I$ of the first temperature measurement element 62 as shown in the above Expression (9). Accordingly, the inventors also have found out that, as shown in the following Expression (23), the calorific value Q per unit volume of the mixed gas is also represented by an equation which has as variables the resistances $R_H(T_{H1})$, $R_H(T_{H2})$, and $R_H(T_{H3})$ of the heater element 61 coming into contact with the mixed gas when the heat generating temperature is $T_{H1}$, $T_{H2}$, and $T_{H3}$ and the resistance $R_I$ of the first temperature measurement element 62 coming into contact with the mixed gas.

$$Q = g[R_H(T_{H1}), R_H(T_{H2}), R_H(T_{H3}), R_I] \qquad \ldots(23)$$

In addition, the heat generating temperature $T_H$ of the heater element 61 depends on the voltage $V_L$ which is applied to the heater element 61. Accordingly, as shown in the following Expression (24), the calorific value Q per unit volume of the mixed gas is represented by an equation which has as variables the resistances $R_H(V_{L1})$, $R_H(V_{L2})$, and $R_H(V_{L3})$ of the heater element 61 coming into contact with the mixed gas when the applied voltage is $V_{L1}$, $V_{L2}$, and $V_{L3}$ and the resistance $R_I$ of the first temperature measurement element 62 coming into contact with the mixed gas.

$$Q = g[R_H(V_{L1}), R_H(V_{L2}), R_H(V_{L3}), R_I] \qquad \ldots(24)$$

Accordingly, the calorific value Q of the examination target mixed gas can be uniquely obtained by measuring and substituting the values of the resistances $R_H(V_{L1})$, $R_H(V_{L2})$, and $R_H(V_{L3})$ of the heater element 61 coming into contact with the examination target mixed gas when the applied voltage is $V_{L1}$, $V_{L2}$, and $V_{L3}$ and the value of the resistance $R_I$ of the first temperature measurement element 62 coming into contact with the examination target mixed gas into Expression (24).

The gas components of the mixed gas are not limited to the four kinds. For example, when the mixed gas has n kinds of gas components, first, an equation which has as variables the resistances $R_H(V_{L1})$, $R_H(V_{L2})$, $R_H(V_{L3})$, ..., $R_H(V_{Ln-1})$ of the heater element 61 to which at least n-1 kinds of voltages $V_{L1}$, $V_{L2}$, $V_{L3}$, ..., $V_{Ln-1}$ are applied and the resistance $R_I$ of the first temperature measurement element 62 is obtained in advance as represented by the following Expression (25). The values of the resistances $R_H(V_{L1})$, $R_H(V_{L2})$, $R_H(V_{L3})$, ..., $R_H(V_{Ln-1})$ of the heater element 61, to which the n-1 kinds of voltages $V_{L1}$, $V_{L2}$, $V_{L3}$, ..., $V_{Ln-1}$ are applied, coming into contact with an examination target mixed gas whose volume ratios of the n kinds of gas components are unknown, and the value of the resistance $R_1$ of the first temperature measurement element 62 coming into contact with the examination target mixed gas are measured and substituted into Expression (25) to uniquely obtain the calorific value Q per unit volume of the examination target mixed gas.

$$Q = g[R_H(V_{L1}), R_H(V_{L2}), R_H(V_{L3}), \ldots, R_H(V_{Ln-1}), R_I] \quad \ldots(25)$$

When the mixed gas includes, in addition to methane ($CH_4$) and propane ($C_3H_8$), alkane ($C_jH_{2j+2}$) other than methane ($CH_4$) and propane ($C_3H_8$) (the symbol j represents a natural number) as a gas component, even when alkane ($C_jH_{2j+2}$) other than methane ($CH_4$) and propane ($C_3H_8$) is regarded as a mixture of methane ($CH_4$) and propane ($C_3H_8$), there is no influence on the calculation of Expression (25). For example, Expression (25) may be calculated regarding ethane ($C_2H_6$), butane ($C_4H_{10}$), pentane ($C_5H_{12}$), and hexane ($C_6H_{12}$) as mixtures of methane ($CH_4$) and propane ($C_3H_8$) multiplied by predetermined coefficients, as represented by the following Expressions (26) to (29).

$$C_2H_6 = 0.5CH_4 + 0.5C_3H_8 \quad \ldots(26)$$

$$C_4H_{10} = -0.5CH_4 + 1.5C_3H_8 \ldots(27)$$

$$C_5H_{12} = -1.0CH_4 + 2.0C_3H_8 \ldots(28)$$

$$C_6H_{14} = -1.5CH_4 + 2.5C_3H_8 \ldots(29)$$

Accordingly, when the mixed gas including the n kinds of gas components includes, as gas components, z kinds (the symbol z represents a natural number) of alkane ($C_jH_{2j+2}$) other than methane ($CH_4$) and propane ($C_3H_8$) in addition to methane ($CH_4$) and propane ($C_3H_8$), an equation which has as variables the resistance $R_H$ of the heater element 61 to which at least n-z-1 kinds of voltages are applied and the resistance $R_I$ of the first temperature measurement element 62 may be obtained.

When the kind of gas components in the mixed gas used to calculate Expression (25) is equal to the kind of gas components in a mixed gas to be examined whose calorific value Q per unit volume is unknown, Expression (25) may be used to calculate the calorific value Q of the mixed gas to be examined. When the mixed gas to be examined includes gas components that are smaller than n kinds of gas components and gas components smaller than n kinds of gas components are included in the mixed gas used to calculate Expression (25), Expression (25) can be used. For example, when the mixed gas used to calculate Expression (25) includes four kinds of gas components, that is, methane ($CH_4$), propane ($C_3H_8$), nitrogen ($N_2$), and carbon dioxide ($CO_2$) and the mixed gas to be examined does not include nitrogen ($N_2$), but includes only three kinds of gas components, that is, methane ($CH_4$), propane ($C_3H_8$), and carbon dioxide ($CO_2$), Expression (25) can also be used to calculate the calorific value Q of the mixed gas to be examined.

When the mixed gas used to calculate Expression (25) includes as gas components methane ($CH_4$) and propane ($C_3H_8$), Expression (25) can be used even when the mixed gas to be examined includes alkane ($C_jH_{2j+2}$) which is not included in the mixed gas used to calculate Expression (25). This is because regarding alkane ($C_jH_{2j+2}$) other than methane ($CH_4$) and propane ($C_3H_8$) as a mixture of methane ($CH_4$) and propane ($C_3H_8$) does not affect the calculation of the calorific value Q per unit volume by Expression (25), as described above.

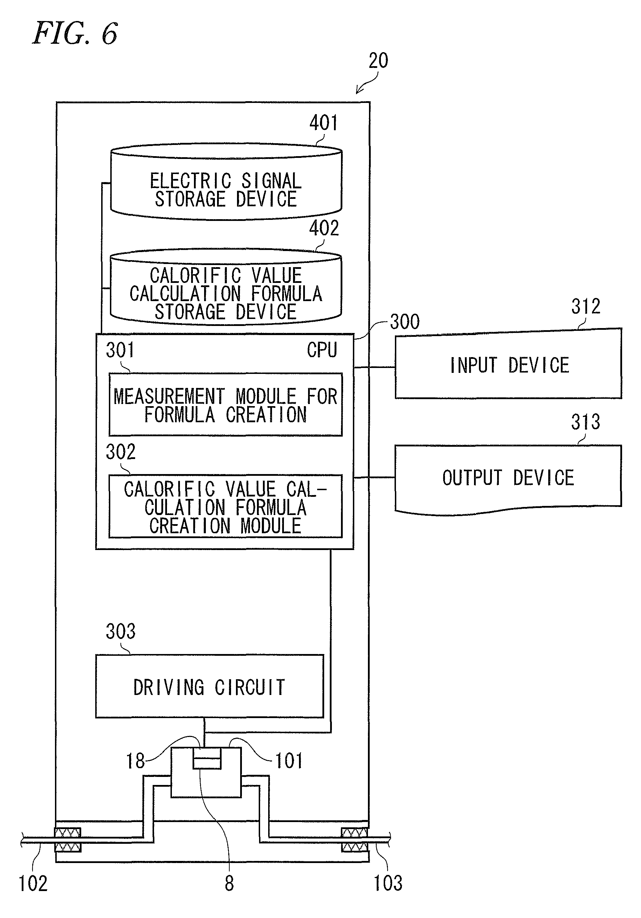

Here, a calorific value calculation formula creation system 20 according to the first embodiment shown in Fig. 6 includes: a chamber 101 which is a container into which a plurality of sample mixed gases are injected; and the microchip 8 which is disposed in the chamber 101 and includes the first temperature measurement element 62 and the heater element 61 to which a plurality of voltages $V_L$ is applied and which generates heat at a plurality of heat generating temperatures $T_H$, which are shown in Fig. 1. Further, the calorific value calculation formula creation system 20 shown in Fig. 6 includes: a measurement module 301 for formula creation, which obtains a measured value indicating the resistance $R_I$ of the first temperature measurement element 62 depending on the temperatures $T_I$ of a plurality of sample mixed gases and a measured value indicating the resistance $R_H$ of the heater element 61 to which a plurality of voltages $V_L$ is applied; and a calorific value calculation formula creation module which creates a calorific value calculation formula, which includes an independent variable indicating the resistance $R_I$ of the first temperature measurement element 62, an independent variable indicating the resistance $R_H$ of the heater element 61 to which a plurality of voltages $V_L$ is applied and a dependent variable indicating the calorific value Q based on the values of the known calorific values Q of a plurality of mixed gases, the measured value indicating the resistance $R_I$ of the first temperature measurement element 62 and the measured value indicating the resistance $R_H$ of the heater element 61 to which a plurality of voltages $V_L$ is applied. The sample mixed gas includes a plurality of kinds of gas components.

The measurement mechanism 10 includes the microchip 8 described with reference to Figs. 1 and 2 which is arranged in the chamber 101 into which the sample mixed gas is injected. The microchip 8 is arranged in the chamber 101 with the insulating member 18 interposed therebetween. A flow path 102 for transferring the sample mixed gas to the chamber 101 and a flow path 103 of exhausting the sample mixed gas from the chamber 101 to the outside are connected to the chamber 101.

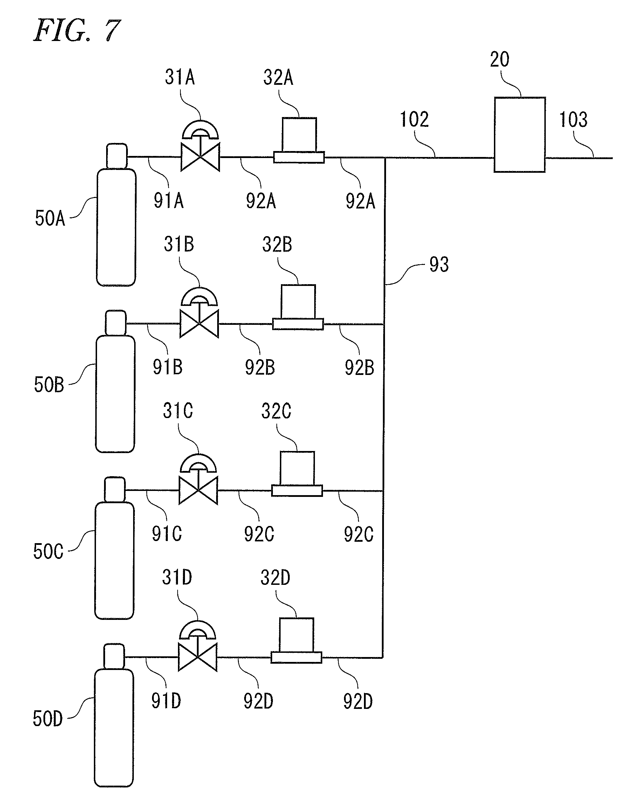

When four kinds of sample mixed gases with different calorific values Q are used, as shown in Fig. 7, a first gas cylinder 50A storing a first sample mixed gas, a second gas cylinder 50B storing a second sample mixed gas, a third gas cylinder 50C storing a third sample mixed gas, and a fourth gas cylinder 50D storing a fourth sample mixed gas are prepared. A first gas pressure adjuster 31A for obtaining the first sample mixed gas which is adjusted to a low pressure of, for example, 0.2 MPa from the first gas cylinder 50A is connected to the first gas cylinder 50A through a flow path 91A. In addition, a first flow rate controller 32A is connected to the first gas pressure adjuster 31A through a flow path 92A. The first flow rate controller 32A controls the flow rate of the first sample mixed gas transferred to the gas property value measurement system 20 through the flow path 92A and a flow path 102.

A second gas pressure adjuster 31B is connected to the second gas cylinder 50B through a flow path 91B. In addition, a second flow rate controller 32B is connected to the second gas pressure adjuster 31B through a flow path 92B. The second flow rate controller 32B controls the flow rate of the second sample mixed gas transferred to the gas property value measurement system 20 through the flow paths 92B, 93, and 102.

A third gas pressure adjuster 31C is connected to the third gas cylinder 50C through a flow path 91 C. In addition, a third flow rate controller 32C is connected to the third gas pressure adjuster 31C through a flow path 92C. The third flow rate controller 32C controls the flow rate of the third sample mixed gas transferred to the gas property value measurement system 20 through the flow paths 92C, 93, and 102.

A fourth gas pressure adjuster 31D is connected to the fourth gas cylinder 50D through a flow path 91D. In addition, a fourth flow rate controller 32D is connected to the fourth gas pressure adjuster 31D through a flow path 92D. The fourth flow rate controller 32D controls the flow rate of the fourth sample mixed gas transferred to the gas property value measurement system 20 through the flow paths 92D, 93, and 102.

Each of the first to fourth sample mixed gases is, for example, a natural gas. Each of the first to fourth sample mixed gases includes, for example, four kinds of gas components, that is, methane ($CH_4$), propane ($C_3H_8$), nitrogen ($N_2$), and carbon dioxide ($CO_2$).

The chamber 101 shown in Fig. 6 is filled with the first sample mixed gas, and then the first temperature measurement element 62 of the microchip 8 shown in Figs. 1 and 2 is supplied with a weak voltage not enough to generate self-heating. Next, the heater element 61 is sequentially supplied with the voltages $V_{L1}$, $V_{L2}$, and $V_{L3}$ from a driving circuit 303 shown in Fig. 6. The first sample mixed gas is removed from the chamber 101 and then the chamber 101 is sequentially filled with the second to fourth sample mixed gases. After the chamber 101 is filled with the second sample mixed gas, the first temperature measurement element 62 of the microchip 8 shown in Figs. 1 and 2 is supplied with a weak voltage not enough to generate self-heating. Next, the heater element 61 coming into contact with the second sample mixed gas is sequentially supplied with the voltages $V_{L1}$, $V_{L2}$, and $V_{L3}$ from the driving circuit 303.

After the chamber 101 shown in Fig. 6 is filled with the third sample mixed gas, the first temperature measurement element 62 of the microchip 8 shown in Figs. 1 and 2 is supplied with a weak voltage not enough to generate self-heating. Next, the heater element 61 coming into contact with the third sample mixed gas is sequentially supplied with the voltages $V_{L1}$, $V_{L2}$, and $V_{L3}$ from the driving circuit 303 shown in Fig. 6. After the chamber 101 shown in Fig. 6 is filled with the fourth sample mixed gas, the first temperature measurement element 62 of the microchip 8 shown in Figs. 1 and 2 is supplied with a weak voltage not enough to generate self-heating. Next, the heater element 61 coming into contact with the fourth sample mixed gas is sequentially supplied with the voltages $V_{L1}$, $V_{L2}$, and $V_{L3}$ from the driving circuit 303 shown in Fig. 6.

When the respective sample mixed gases include n kinds of gas components, the heater element 61 of the microchip 8 shown in Figs. 1 and 2 is supplied with at least n-1 kinds of different voltages. However, as described above, alkane ($C_jH_{2j+2}$) other than methane ($CH_4$) and propane ($C_3H_8$) can be regarded as a mixture of methane ($CH_4$) and propane ($C_3H_8$). Accordingly, when the sample mixed gas including n kinds of gas components includes, as gas components, z kinds (the symbol z represents a natural number) of alkane ($C_jH_{2j+2}$) in addition to methane ($CH_4$) and propane ($C_3H_8$), the heater element 61 is supplied with at least (n - z - 1) kinds of different voltages.

As shown in Fig. 6, the microchip 8 is connected to a central processing unit (CPU) 300 including the measurement module 301. An electric signal storage device 401 is connected to the CPU 300. The measurement module 301 measures the value of the resistance $R_I$ of the first temperature measurement element 62 to which a weak voltage not enough to generate self-heating is applied and the values of the resistances $R_H(V_{L1})$, $R_H(V_{L2})$, and $R_H(V_{L3})$ of the heater element 61 to which the voltages $V_{L1}$, $V_{L2}$, and $V_{L3}$ are applied, and saves the measured values in the electric signal storage device 401.

The calorific value calculation formula creation module 302 included in the CPU 300 collects the values of the known calorific values Q of, for example, the first to fourth sample mixed gases, a plurality of measured values of the resistance $R_I$ of the first temperature measurement element 62 and a plurality of measured values of the resistances $R_H(V_{L1})$, $R_H(V_{L2})$, and $R_H(V_{L3})$ of the heater element 61. Further, the calorific value calculation formula creation module 302 calculates a calorific value calculation formula, which has the resistance $R_I$ of the first temperature measurement element 62 and the resistances $R_H(V_{L1})$, $R_H(V_{L2})$, and $R_H(V_{L3})$ of the heater element 61 as independent variables and the calorific value Q as a dependent variable, by using multiple classification analysis based on the collected values of the calorific values Q, the resistance $R_I$ of the first temperature measurement element 62 and the resistances $R_H(V_{L1})$, $R_H(V_{L2})$, and $R_H(V_{L3})$ of the heater element 61.

The "multivariate analysis" includes a support vector regression and multiple regression analysis disclosed in A.J. Smola and B. Scholkopf, "A Tutorial on Support Vector Regression" (NeuroCOLT Technical Report (NC-TR-98-030), 1998) and Fuzzy Qualification Theory of Second Order disclosed in JP-A-5-141999.

The calorific value calculation formula creation system 20 further includes a calorific value calculation formula storage device 402 connected to the CPU 300. The calorific value calculation formula storage device 402 saves the calorific value calculation formula created by the calorific value calculation formula creation module 302. Further, an input device 312 and an output device 313 are connected to the CPU 300. As the input device 312, for example, a keyboard, a pointing device such as a mouse, or the like can be used. As the output device 313, an image display device such as a liquid crystal display or a monitor, a printer, or the like can be used.

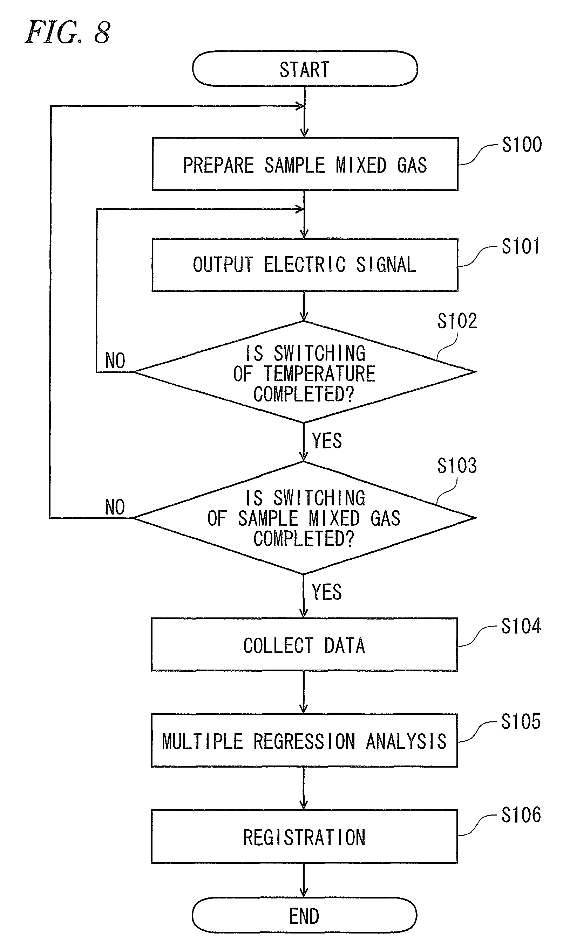

Next, a method of creating a calorific value calculation formula according to the first embodiment will be described using a flowchart shown in Fig. 8.

(a) In Step S100, the heat-retaining element 64 shown in Figs. 1 and 2 is allowed to generate heat to constantly maintain the temperature of the substrate 60 to, for example, 60°C. Next, a valve for a first flow rate controller 32A is opened while keeping valves for second to fourth flow rate controllers 32B to 32D closed, and the first sample mixed gas is introduced into the chamber 101 shown in Fig. 6. In Step S101, the measurement module 301 measures the value of the resistance $R_I$ of the first temperature measurement element 62 coming into contact with the first sample mixed gas and saves in the electric signal storage device 401. Next, the driving circuit 303 applies the voltage $V_{L1}$ to the heater element 61 shown in Figs. 1 and 2 to allow the heater element 61 to generate heat. The measurement module 301 shown in Fig. 6 saves the value of the resistance $R_H(V_{L1})$ of the heater element 61, coming into contact with the first sample mixed gas, to which the voltage $V_{L1}$ is applied in the electric signal storage device 401.

(b) In Step S102, the driving circuit 303 determines whether or not the switching of the voltage $V_L$ which is applied to the heater element 61 shown in Figs. 1 and 2 is completed. When the switching to the voltages $V_{L2}$ and $V_{L3}$ is not completed, the process returns to Step S101 and the driving circuit 303 shown in Fig. 6 applies the voltage $V_{L2}$ to the heater element 61 shown in Figs. 1 and 2 to allow the heater element 61 to generate heat. The measurement module 301 shown in Fig. 6 saves the value of the resistance $R_H(V_{L2})$ of the heater element 61, coming into contact with the first sample mixed gas, to which the voltage $V_{L2}$ is applied in the electric signal storage device 401.

(c) It is again determined whether or not the switching of the voltage $V_L$ which is applied to the heater element 61 shown in Figs. 1 and 2 is completed in Step S102. When the switching to the voltage $V_{L3}$ is not completed, the process returns to Step S101 and the driving circuit 303 shown in Fig. 6 applies the voltage $V_{L3}$ to the heater element 61 shown in Figs. 1 and 2 to allow the heater element 61 to generate heat. The measurement module 301 shown in Fig. 6 saves the value of the resistance $R_H(V_{L3})$ of the heater element 61, coming into contact with the first sample mixed gas, to which the voltage $V_{L3}$ is applied in the electric signal storage device 401.

(d) When the switching to the voltage $V_L$ which is applied to the heater element 61 is completed, the process advances to Step S103 from Step S102. In Step S103, it is determined whether or not the switching of the sample mixed gas is completed. When the switching to the second to fourth sample mixed gases is not completed, the process returns to Step S100. In Step S100, the first flow rate controller 32A shown in Fig. 7 is closed, the valve for the second flow rate controller 32B is opened while maintaining the closed states of the valves for the third and fourth flow rate controllers 32C and 32D, and the second sample mixed gas is introduced into the chamber 101 shown in Fig. 6.

(e) As in the case of the first sample mixed gas, the loop of Step S101 and Step S102 is repeatedly performed. The measurement module 301 measures the value of the resistance $R_I$ of the first temperature measurement element 62 coming into contact with the second sample mixed gas and saves in the electric signal storage device 401. In addition, the measurement module 301 saves the values of the resistances $R_H(V_{L1})$, $R_H(V_{L2})$, and $R_H(V_{L3})$ of the heater element 61, coming into contact with the second sample mixed gas, to which the voltages $V_{L1}$, $V_{L2}$, and $V_{L3}$

are applied in the electric signal storage device 401. After that, the loop of Steps S 100 to Step S103 is repeated. Therefore, the value of the resistance $R_I$ of the first temperature measurement element 62 coming into contact with the third sample mixed gas, the values of the resistances $R_H(V_{L1})$, $R_H(V_{L2})$, and $R_H(V_{L3})$ of the heater element 61, coming into contact with the third sample mixed gas, to which the voltages $V_{L1}$, $V_{L2}$, and $V_{L3}$ are applied, the value of the resistance $R_I$ of the first temperature measurement element 62 coming into contact with the fourth sample mixed gas, and the values of the resistances $R_H(V_{L1})$, $R_H(V_{L2})$, and $R_H(V_{L3})$ of the heater element 61, coming into contact with the fourth sample mixed gas, to which the voltages $V_{L1}$, $V_{L2}$, and $V_{L3}$ are applied are saved in the electric signal storage device 401.

(f) In Step S104, the value of the known calorific value Q of the first sample mixed gas, the value of the known calorific value Q of the second sample mixed gas, the value of the known calorific value Q of the third sample mixed gas and the value of the known calorific value Q of the fourth sample mixed gas are input to the calorific value calculation formula creation module 302 from the input device 312. In addition, the calorific value calculation formula creation module 302 reads out a plurality of the measured values of the resistance $R_I$ of the first temperature measurement element 62 and a plurality of measured values of the resistances $R_H(V_{L1})$, $R_H(V_{L2})$, and $R_H(V_{L3})$ of the heater element 61 from the electric signal storage device 401.

(g) In Step S105, the calorific value calculation formula creation module 302 performs multiple regression analysis based on the values of the calorific values Q of the first to fourth sample mixed gases, a plurality of the measured values of the resistance $R_I$ of the first temperature measurement element 62 and a plurality of measured values of the resistances $R_H(V_{L1})$, $R_H(V_{L2})$, and $R_H(V_{L3})$ of the heater element 61. By the multiple regression analysis, the calorific value calculation formula creation module 302 calculates a calorific value calculation formula which has the resistance $R_I$ of the first temperature measurement element 62 and the resistances $R_H(V_{L1})$, $R_H(V_{L2})$, and $R_H(V_{L3})$ of the heater element 61 as independent variables and the calorific value Q as a dependent variable. After that, in Step S106, the calorific value calculation formula creation module 302 saves the created calorific value calculation formula in the calorific value calculation formula storage device 402 and the method of creating a calorific value calculation formula according to the first embodiment ends.

As described above, according to the method of creating a calorific value calculation formula according to the first embodiment, it is possible to create a calorific value calculation formula which can uniquely calculate the value of the calorific value Q of a measurement target mixed gas.

(Second Embodiment)

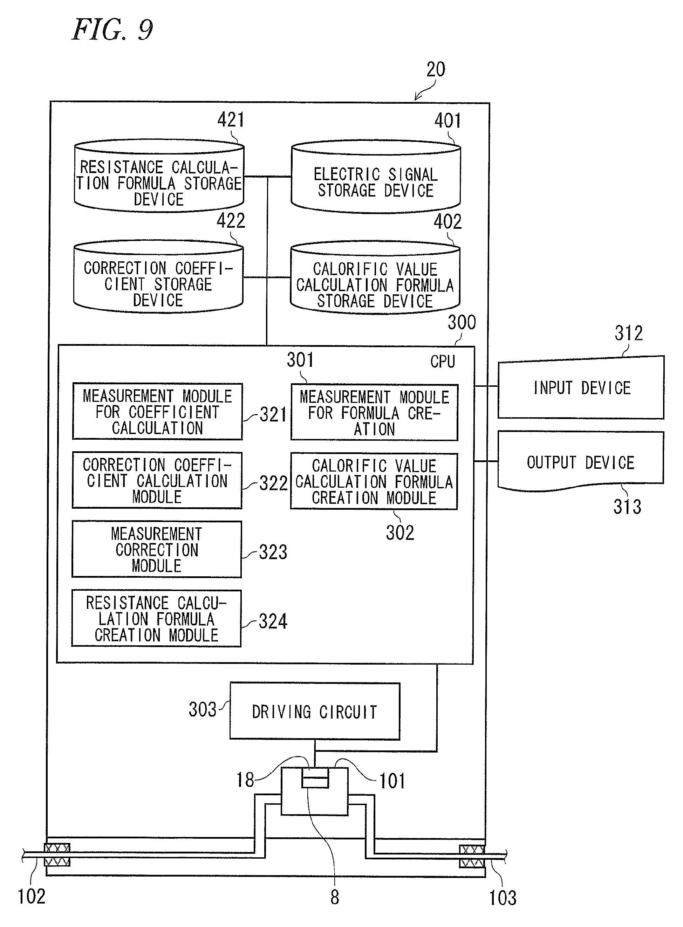

As shown in Fig. 9, a calorific value calculation formula creation system 20 according to a second embodiment includes: a resistance calculation formula storage device 421 which saves a resistance calculation formula having an independent variable indicating the temperature $T_I$ detected by the first temperature measurement element 62, coming into contact with a calibration gas, shown in Figs. 1 and 2 and a dependent variable indicating the resistance $R_H$ of the heater element 61 coming into contact with the calibration gas; a measurement module 321 for coefficient calculation, which obtains the measured value indicating the temperature $T_I$ detected by the first temperature measurement element 62 coming into contact with the calibration gas and the measured value indicating the resistance $R_H$ of the heater element 61 coming into contact with the calibration gas; and a correction coefficient calculation module 322 which calculates a calculated value indicating the resistance $R_H$ of the heater element 61 by substituting the measured value indicating the temperature $T_I$ into the independent variable indicating the temperature $T_I$ of the resistance calculation formula and calculates a correction coefficient which is a ratio between the measured value indicating the resistance $R_H$ of the heater element 61 and the calculated value indicating the resistance $R_H$ of the heater element 61.

Here, a method of obtaining a resistance calculation formula will be described. First, the heat-retaining element 64 is allowed to generate heat to maintain the temperature of the substrate 60 to a certain temperature, for example, 60°C. Hereinafter, the temperature of the substrate 60 is denoted by $T_S$. Next, a methane gas at -10°C as a calibration gas is injected into a chamber 101 shown in Fig. 9. At this time, an atmospheric temperature $T_{I1}$ which is detected by the first temperature measurement element 62 is measured by the measurement module 301. The atmospheric temperature $T_{I1}$ is, for example, 58°C. Next, the heater element 61 is sequentially supplied with the voltages $V_{L1}$, $V_{L2}$, and $V_{L3}$ and the values of the resistances $R_{H\_Ch\_TI1}(V_{L1})$, $R_{H\_Ch\_TI1}(V_{L2})$, and $R_{H\_Ch\_TI1}(V_{L3})$ of the heater element 61 are measured by the measurement module 301. After that, the methane gas is discharged.

Next, a methane gas at 5°C is injected into the chamber 101. At this time, an atmospheric temperature $T_{I2}$ which is detected by the first temperature measurement element 62 is measured by the measurement module 301. The atmospheric temperature $T_{I2}$ is, for example, 59°C. Next, the heater element 61 is sequentially supplied with the voltages $V_{L1}$, $V_{L2}$, and $V_{L3}$ and the values of the resistances $R_{H\_Ch\_TI2}(V_{L1})$, $R_{H\_Ch\_TI2}(V_{L2})$, and $R_{H\_Ch\_TI2}(V_{L3})$ of the heater element 61 are measured by the measurement module 301. After that, the methane gas is discharged.

Next, a methane gas at 23°C is injected into the chamber 101. At this time, an atmospheric temperature $T_{I3}$

which is detected by the first temperature measurement element 62 is measured by the measurement module 301. The atmospheric temperature $T_{I3}$ is, for example, 60°C and is equal to the temperature $T_S$ of the substrate 60. Next, the heater element 61 is sequentially supplied with the voltages $V_{L1}$, $V_{L2}$, and $V_{L3}$ and the values of the resistances $R_{H\_Ch\_TI3}(V_{L1})$, $R_{H\_Ch\_TI3}(V_{L2})$, and $R_{H\_Ch\_TI3}(V_{L3})$ of the heater element 61 are measured by the measurement module 301. After that, the methane gas is discharged.

Next, a methane gas at 40°C is injected into the chamber 101. At this time, an atmospheric temperature $T_{I4}$ which is detected by the first temperature measurement element 62 is measured by the measurement module 301. The atmospheric temperature $T_{I4}$ is, for example, 61 °C. Next, the heater element 61 is sequentially supplied with the voltages $V_{L1}$, $V_{L2}$, and $V_{L3}$ and the values of the resistances $R_{H\_Ch\_TI4}(V_{L1})$, $R_{H\_Ch\_TI4}(V_{L2})$, and $R_{H\_Ch\_TI4}(V_{L3})$ of the heater element 61 are measured by the measurement module 301. After that, the methane gas is discharged.

Next, a methane gas at 50°C is injected into the chamber 101. At this time, an atmospheric temperature $T_{I5}$ which is detected by the first temperature measurement element 62 is measured by the measurement module 301. The atmospheric temperature $T_{I5}$ is, for example, 62°C. Next, the heater element 61 is sequentially supplied with the voltages $V_{L1}$, $V_{L2}$, and $V_{L3}$ and the values of the resistances $R_{H\_Ch\_TI5}(V_{L1})$, $R_{H\_Ch\_TI5}(V_{L2})$, and $R_{H\_Ch\_TI5}(V_{L3})$ of the heater element 61 are measured by the measurement module 301. After that, the methane gas is discharged.

The CPU 300 further includes a resistance calculation formula creation module 324. The resistance calculation formula creation module 324 collects the values of the atmospheric temperatures $T_{I1}$, $T_{I2}$, $T_{I3}$, $T_{I4}$, and $T_{I5}$, and the values of the resistances $R_{H\_Ch\_TI1}(V_{L1})$, $R_{H\_Ch\_TI2}(V_{L1})$, $R_{H\_Ch\_TI3}(V_{L1})$, $R_{H\_Ch\_TI4}(V_{L1})$, and $R_{H\_Ch\_TI5}(V_{L1})$ of the heater element 61 to which the voltage $V_{L1}$ is applied. Here, the value of the resistance $R_{H\_Ch\_TI3}(V_{L1})$ when the atmospheric temperature $T_{I3}$ which is detected by the first temperature measurement element 62 becomes equal to the temperature $T_S$ of the substrate 60 is $R_{H\_Ch\_Ts}(V_{L1})$, and the resistance calculation formula creation module 324 creates a resistance calculation formula which has the atmospheric temperature $T_I$ detected by the first temperature measurement element 62 as an independent variable and the resistance $R_{H\_Ch}(V_{L1})$ of the heater element 61 to which the voltage $V_{L1}$ is applied as a dependent variable.

$$R_{H\_Ch}(V_{L1}) = R_{H\_Ch\_Ts}(V_{L1})[1 + a_{L1}(T_I - T_S)] \qquad \ldots (30)$$

where the symbol $a_{L1}$ represents a constant. When the temperature of the substrate 60 is maintained at 60°C, the following Expression (31) is obtained from the above Expression (30).

$$R_{H\_Ch}(V_{L1}) = R_{H\_Ch\_Ts}(V_{L1})[1 + a_{L1}(T_I - 60)] \qquad \ldots (31)$$

In addition, the resistance calculation formula creation module 324 collects the values of the atmospheric temperatures $T_{I1}$, $T_{I2}$, $T_{I3}$, $T_{I4}$, and $T_{I5}$, and the values of the resistances $R_{H\_Ch\_TI1}(V_{L2})$, $R_{H\_Ch\_TI2}(V_{L2})$, $R_{H\_Ch\_TI3}(V_{L2})$, $R_{H\_Ch\_TI4}(V_{L2})$, and $R_{H\_Ch-TI5}(V_{L2})$ of the heater element 61 to which the voltage $V_{L2}$ is applied. Here, the value of the resistance $R_{H\_Ch\_TI3}(V_{L2})$ when the atmospheric temperature $T_{I3}$ which is detected by the first temperature measurement element 62 becomes equal to the temperature $T_S$ of the substrate 60 is $R_{H\_Ch\_Ts}(V_{L2})$, and the resistance calculation formula creation module 324 creates a resistance calculation formula which has the atmospheric temperature $T_I$ detected by the first temperature measurement element 62 as an independent variable and the resistance $R_{H\_Ch}(V_{L2})$ of the heater element 61 to which the voltage $V_{L2}$ is applied as a dependent variable.

$$R_{H\_Ch}(V_{L2}) = R_{H\_Ch\_Ts}(V_{L2})[1 + a_{L2}(T_I - T_S)] \qquad \ldots (32)$$

where the symbol $a_{L2}$ represents a constant. When the temperature of the substrate 60 is maintained at 60°C, the following Expression (33) is obtained from the above Expression (32).

$$R_{H\_Ch}(V_{L2}) = R_{H\_Ch\_Ts}(V_{L2})[1 + a_{L2}(T_I - 60)] \qquad \ldots (33)$$

In addition, the resistance calculation formula creation module 324 collects the values of the atmospheric temperatures $T_{I1}$, $T_{I2}$, $T_{I3}$, $T_{I4}$, and $T_{I5}$, and the values of the resistances $R_{H\_Ch\_TI1}(V_{L3})$, $R_{H\_Ch\_TI2}(V_{L3})$, $R_{H\_Ch\_TI3}(V_{L3})$, $R_{H\_Ch\_TI4}(V_{L3})$, and $R_{H\_Ch\_TI5}(V_{L3})$ of the heater element 61 to which the voltage $V_{L3}$ is applied. Here, the value of the resistance $R_{H\_Ch\_TI3}(V_{L3})$ when the atmospheric temperature $T_{I3}$ which is detected by the first temperature measurement element 62 becomes equal to the temperature $T_S$ of the substrate 60 is $R_{H\_Ch\_Ts}(V_{L3})$, and the resistance calculation formula creation module 324 creates a resistance calculation formula which has the atmospheric temperature $T_I$ detected by the first temperature measurement element 62 as an independent variable and the resistance $R_{H\_Ch}(V_{L3})$ of the heater element 61 to which the voltage $V_{L3}$ is applied as a dependent variable.

$$R_{H\_Ch}(V_{L3}) = R_{H\_Ch\_Ts}(V_{L3})[1 + a_{L3}(T_I - T_S)] \qquad \ldots(34)$$

where the symbol $a_{L3}$ represents a constant. When the temperature of the substrate 60 is maintained at 60°C, the following Expression (35) is derived from the above Expression (34).

$$R_{H\_Ch}(V_{L3}) = R_{H\_Ch\_Ts}(V_{L3})[1 + a_{L3}(T_I - 60)] \qquad \ldots(35)$$

The resistance calculation formula creation module 324 saves the above-described calculated Expressions (31), (33), and (35) as resistance calculation formulas in the resistance calculation formula storage device 421. Here, since a high voltage is applied to the heater element 61 shown in Figs. 1 and 2 so that the above element generates heat at a high temperature, electromigration may occur. Accordingly, in some cases, the value of the resistance $R_H$ of the heater element 61 to which a predetermined voltage $V_L$ is applied under a predetermined atmospheric temperature drifts from the initial value due to changes over time and the like. The resistance calculation formula is created before the occurrence of electromigration in the heater element 61, for example, when the calorific value calculation formula creation system 20 shown in Fig. 9 is manufactured, and is saved in the resistance calculation formula storage device 421. However, the time to create the resistance calculation formula is not limited thereto.

Next, for example, the calorific value calculation formula creation system 20 shown in Fig. 9 is operated for a certain period of time in which the electromigration may occur in the heater element 61, and then the heat-retaining element 64 shown in Figs. 1 and 2 is allowed to generate heat to constantly maintain the temperature of the substrate 60 to, for example, 60°C. Further, a methane gas of an arbitrary temperature is injected into the chamber 101 shown in Fig. 9. At this time, the value of the atmospheric temperature $T_I$ which is detected by the first temperature measurement element 62 is measured by the measurement module 321. In addition, the heater element 61 is sequentially supplied with the voltages $V_{L1}$, $V_{L2}$, and $V_{L3}$ and the values of the resistances $R_H(V_{L1})$, $R_H(V_{L2})$, and $R_H(V_{L3})$ of the heater element 61 are measured by the measurement module 321. After that, the methane gas is discharged.

The correction coefficient calculation module 322 substitutes the measured value into the variable which is the atmospheric temperature $T_I$ detected by the first temperature measurement element 62, which is included in the above Expressions (31), (33), and (35), to calculate the values of the resistances $R_{H\_Ch}(V_{L1})$, $R_{H\_Ch}(V_{L2})$, and $R_{H\_Ch}(V_{L3})$ of the heater element 61 to which the voltages $V_{L1}$, $V_{L2}$, and $V_{L3}$ are applied. Further, as shown in the following Expression (36), the correction coefficient calculation module 322 divides the calculated value $R_{H\_Ch}(V_{L1})$ of the resistance of the heater element 61 supplied with the voltage $V_{L1}$ by the measured value $R_H(V_{L1})$ to calculate a correction coefficient $cf(V_{L1})$.

$$cf(V_{L1}) = R_{H\_Ch}(V_{L1})/R_H(V_{L1}) \qquad \ldots(36)$$

Here, the correction coefficient $cf(V_{L1})$ represents a degree of the drift of the resistance of the heater element 61 supplied with the voltage $V_{L1}$, which is generated from when Expression (31) is created to when the value of the resistance $R_H(V_{L1})$ of the heater element 61 supplied with the voltage $V_{L1}$ is measured by the measurement module 321. For example, when the drift is not generated, the value of the correction coefficient $cf(V_{L1})$ is 1.

In addition, as shown in the following Expression (37), the correction coefficient calculation module 322 divides the calculated value $R_{H\_Ch}(V_{L2})$ of the resistance of the heater element 61 supplied with the voltage $V_{L2}$ by the measured value $R_H(V_{L2})$ to calculate a correction coefficient $cf(V_{L2})$.

$$cf(V_{L2}) = R_{H\_Ch}(V_{L2})/R_H(V_{L2}) \qquad \ldots(37)$$

Here, the correction coefficient $cf(V_{L2})$ represents a degree of the drift of the resistance of the heater element 61 supplied with the voltage $V_{L2}$, which is generated from when Expression (33) is created to when the value of the resistance $R_H(V_{L2})$ of the heater element 61 supplied with the voltage $V_{L2}$ is measured by the measurement module 321.

Further, as shown in the following Expression (38), the correction coefficient calculation module 322 divides the calculated value $R_{H\_Ch}(V_{L3})$ of the resistance of the heater element 61 supplied with the voltage $V_{L3}$ by the measured value $R_H(V_{L3})$ to calculate a correction coefficient $cf(V_{L3})$.

$$\mathrm{cf}(V_{L3}) = R_{H\_Ch}(V_{L3})/R_H(V_{L3}) \qquad \ldots(38)$$

Here, the correction coefficient $\mathrm{cf}(V_{L3})$ represents a degree of the drift of the resistance of the heater element 61 supplied with the voltage $V_{L3}$, which is generated from when Expression (35) is created to when the value of the resistance $R_H(V_{L3})$ of the heater element 61 supplied with the voltage $V_{L3}$ is measured by the measurement module 321.

The correction coefficient calculation module 322 saves the calculated correction coefficients $\mathrm{cf}(V_{L1})$, $\mathrm{cf}(V_{L2})$ and $\mathrm{cf}(V_{L3})$ in a correction coefficient storage device 422 connected to the CPU 300.

The calorific value calculation formula creation system 20 according to the second embodiment further includes a measurement correction module 323 which corrects, by using the correction coefficients $\mathrm{cf}(V_{L1})$, $\mathrm{cf}(V_{L2})$, and $\mathrm{cf}(V_{L3})$, the measured values indicating the resistances $R_H(V_{L1})$, $R_H(V_{L2})$, and $R_H(V_{L3})$ of the heater element 61 coming into contact with the respective mixed gases, which are measured by the measurement module 301.

In greater detail, as shown in the following Expression (39), the measurement correction module 323 multiplies the measured value of the resistance $R_H(V_{L1})$ of the heater element 61 by the correction coefficient $\mathrm{cf}(V_{L1})$ to calculate a corrected measured value $R_{H\_CR}(V_{L1})$ of the resistance of the heater element 61. In addition, as shown in the following Expression (40), the measurement correction module 323 multiplies the measured value of the resistance $R_H(V_{L2})$ of the heater element 61 by the correction coefficient $\mathrm{cf}(V_{L2})$ to calculate a corrected measured value $R_{H\_CR}(V_{L2})$ of the resistance of the heater element 61. Further, as shown in the following Expression (41), the measurement correction module 323 multiplies the measured value of the resistance $R_H(V_{L3})$ of the heater element 61 by the correction coefficient $\mathrm{cf}(V_{L3})$ to calculate a corrected measured value $R_{H\_CR}(V_{L3})$ of the resistance of the heater element 61.

$$R_{H\_CR}(V_{L1}) = \mathrm{cf}(V_{L1}) \times R_H(V_{L1}) \qquad \ldots(39)$$

$$R_{H\_CR}(V_{L2}) = \mathrm{cf}(V_{L2}) \times R_H(V_{L2}) \qquad \ldots(40)$$

$$R_{H\_CR}(V_{L3}) = \mathrm{cf}(V_{L3}) \times R_H(V_{L3}) \qquad \ldots(41)$$

In the second embodiment, the calorific value calculation formula creation module 302 collects the values of the known calorific values Q of, for example, the first to fourth sample mixed gases, a plurality of measured values of the resistance $R_I$ of the first temperature measurement element 62 and a plurality of corrected measured values $R_{H\_CR}(V_{L1})$, $R_{H\_CR}(V_{L2})$, and $R_{H\_CR}(V_{L3})$ of the resistances of the heater element 61. Further, the calorific value calculation formula creation module 302 calculates a calorific value calculation formula, which has the resistance $R_I$ of the first temperature measurement element 62 and the resistances $R_H(V_{L1})$, $R_H(V_{L2})$, and $R_H(V_{L3})$ of the heater element 61 as independent variables and the calorific value Q as a dependent variable, by using multiple classification analysis based on the collected values of the calorific values Q, value of the resistance $R_I$ of the first temperature measurement element 62 and corrected measured values $R_{H\_CR}(V_{L1})$, $R_{H\_CR}(V_{L2})$, and $R_{H\_CR}(V_{L3})$ of the heater element 61.

Since the other constituent elements in the calorific value calculation formula creation system 20 according to the second embodiment are the same as those in the first embodiment, a description thereof will be omitted herein. According to the calorific value calculation formula creation system 20 of the second embodiment, a variation in the resistance value due to the electromigration which may occur in the heater element 61 can be corrected. Accordingly, it is possible to create the calorific value calculation formula with high accuracy. In the above Expressions (31), (33), and (35), in place of the independent variable which is the temperature $T_I$, an independent variable which is the resistance $R_I$ of the first temperature measurement element 62 depending on the temperature $T_I$ may be included. Accordingly, in this disclosure, the independent variable indicating the temperature $T_I$ which is detected by the first temperature measurement element 62 means both the independent variable which is the temperature $T_I$ itself detected by the first temperature measurement element 62 and the independent variable which is the resistance $R_I$ of the first temperature measurement element 62 reflecting the temperature $T_I$.

(Third Embodiment)

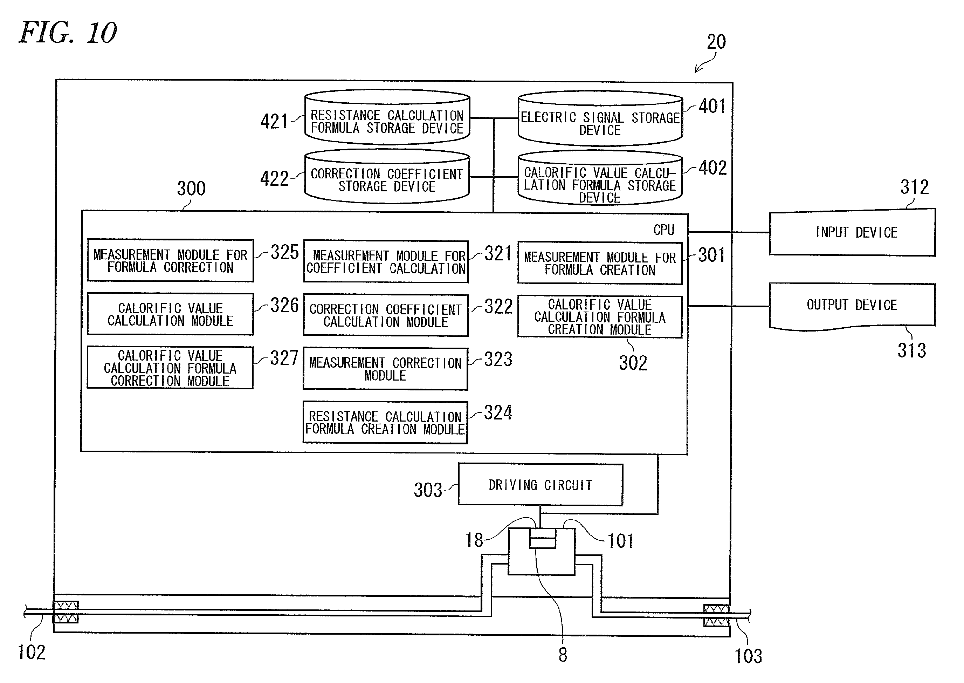

As shown in Fig. 10, a calorific value calculation formula creation system 20 according to a third embodiment includes: a measurement module 325 for formula correction, which obtains the measured value indicating the resistance $R_I$ of the first temperature measurement element 62 coming into contact with a calibration gas and the measured value indicating the resistance $R_H$ of the heater element 61 coming into contact with the calibration gas; a calorific value calculation module 326 which substitutes the measured value indicating the resistance $R_I$ of the first temperature measurement element 62 coming into contact with the calibration gas and the measured value $R_{H\_CR}$, corrected with a correction coefficient cf, indicating the resistance $R_H$ of the heater element 61 coming into contact with the calibration gas, into the independent variable indicating the resistance $R_I$ of the first temperature measurement element 62 and the independent variable indicating the resistance $R_H$ of the heater element 61 in the calorific value calculation formula to obtain a calculated value of the calorific value of the calibration gas; and a calorific value calculation formula correction module 327 which corrects the calorific value calculation formula so as to eliminate the difference between a predetermined value, obtained in advance, of the calorific value of the calibration gas and a calculated value of the calorific value of the calibration gas.

After creation of the calorific value calculation formula by the calorific value calculation formula creation module 302, the measurement module 325 obtains the measured value of the resistance $R_I$ of the first temperature measurement element 62 coming into contact with a calibration gas, for example, methane, and the measured values of the resistances $R_H(V_{L1})$, $R_H(V_{L2})$, and $R_H(V_{L3})$ of the heater element 61 to which voltages $V_{L1}$, $V_{L2}$, and $V_{L3}$ are sequentially applied. The measurement correction module 323 multiplies the measured values of the resistances $R_H(V_{L1})$, $R_H(V_{L2})$, and $R_H(V_{L3})$ of the heater element 61 by the correction coefficients $cf(V_{L1})$, $cf(V_{L2})$, and $cf(V_{L3})$, respectively, to calculate the corrected measured values $R_{H\_CR}(V_{L1})$, $R_{H\_CR}(V_{L2})$, and $R_{H\_CR}(V_{L3})$ of the resistances of the heater element 61.

The calorific value calculation module 326 substitutes the measured value of the resistance $R_I$ of the first temperature measurement element 62 coming into contact with methane into the independent variable which is the resistance $R_I$ of the first temperature measurement element 62 in the calorific value calculation formula. In addition, the calorific value calculation module 326 substitutes the corrected measured values $R_{H\_CR}(V_{L1})$, $R_{H\_CR}(V_{L2})$, and $R_{H\_CR}(V_{L3})$ of the resistances of the heater element 61 coming into contact with methane into the independent variables which are the resistances $R_H(V_{L1})$, $R_H(V_{L2})$, and $R_H(V_{L3})$ of the heater element 61 in the calorific value calculation formula to calculate the value of the calorific value Q of the methane gas.

The calorific value calculation formula correction module 327 compares a theoretical value 37.78 MJ/m$^3$ as the predetermined value, obtained in advance, of the calorific value Q of the methane gas with the calculated value of the calorific value Q of the methane gas calculated by the calorific value calculation module 326. When there is a difference between the theoretical value and the calculated value of the calorific value Q of the methane gas, the calorific value calculation formula correction module 327 corrects the calorific value calculation formula by applying a coefficient, which is obtained by dividing the theoretical value of the calorific value Q of the methane gas by the calculated value, to the calorific value calculation formula. The calorific value calculation formula correction module 327 saves the corrected calorific value calculation formula in the calorific value calculation formula storage device 402.

Since the other constituent elements in the calorific value calculation formula creation system 20 according to the third embodiment are the same as those in the second embodiment, a description thereof will be omitted. It is possible to calculate the calorific value with higher accuracy by using the calorific value calculation formula which is created and corrected by the calorific value calculation formula creation system 20 according to the third embodiment.

(Fourth Embodiment)

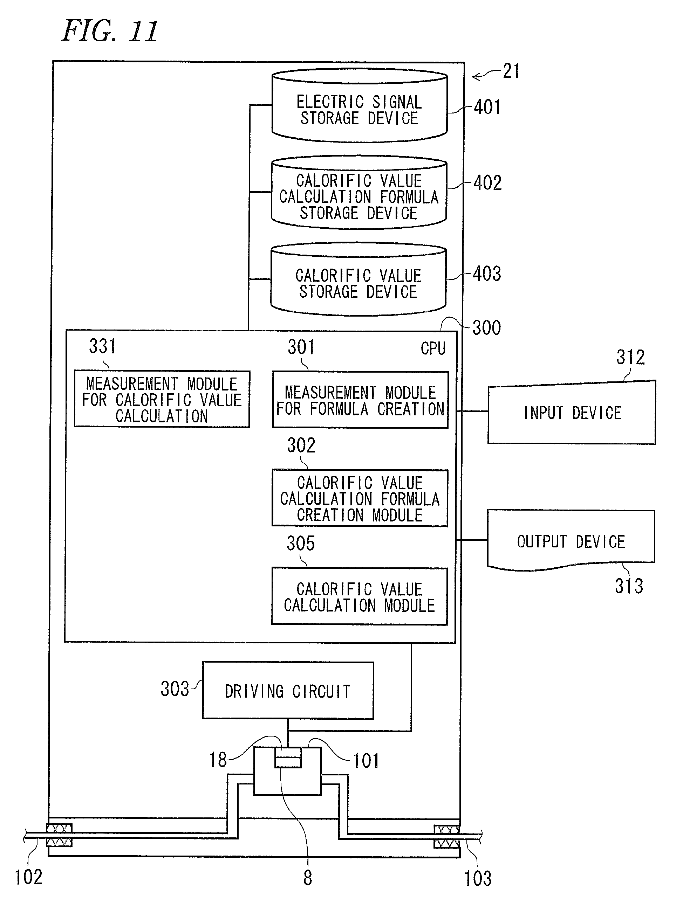

As shown in Fig. 11, a calorific value measurement system 21 according to a fourth embodiment includes: a chamber 101 into which a measurement target mixed gas having an unknown calorific value Q is injected; and a microchip 8 which is disposed in the chamber 101 and includes the first temperature measurement element 62 and the heater element 61 to which a plurality of voltages $V_L$ is applied, which are shown in Figs. 1 and 2. The calorific value measurement system 21 shown in Fig. 11 further includes: a measurement module 331 for calorific value calculation which obtains a measured value indicating the resistance $R_I$ of the first temperature measurement element 62 depending on the temperatures $T_I$ of the measurement target mixed gas and measured values indicating the resistances $R_H$ of the heater element 61 to which a plurality of voltages $V_L$ is applied; a calorific value calculation formula storage device 402 which saves a calorific value calculation formula having an independent variable indicating the resistance $R_I$ of the first temperature measurement element 62, independent variables indicating the resistances $R_H$ of the heater element 61 to which a plurality of voltages $V_L$ is applied and a dependent variable which is the calorific value Q; and a calorific value calculation module which substitutes the measured value indicating the resistance $R_I$ of the first temperature measurement element 62 and the measured value of the resistance $R_H$ of the heater element 61 into the independent variable indicating the resistance $R_I$ of the first temperature measurement element 62 and the independent variable indicating the resistance $R_H$ of the heater element 61 in the calorific value calculation formula to calculate the value of the calorific value Q of the measurement target mixed gas.

The calorific value calculation formula storage device 402 saves the calorific value calculation formula described in the first to third embodiments. Here, a case will be described in which, for example, a natural gas including methane ($CH_4$), propane ($C_3H_8$), nitrogen ($N_2$), and carbon dioxide ($CO_2$) is used as a sample mixed gas in order to create the calorific value calculation formula. In addition, the calorific value calculation formula has, as independent variables, the resistance $R_I$ of the first temperature measurement element 62, the resistance $R_H(V_{L1})$ of the heater element 61 to which the voltage $V_{L1}$ is applied, the resistance $R_H(V_{L2})$ of the heater element 61 to which the voltage $V_{L2}$ is applied, and the resistance $R_H(V_{L3})$ of the heater element 61 to which the voltage $V_{L3}$ is applied.

In the fourth embodiment, for example, a natural gas as a measurement target mixed gas having an unknown calorific value Q which includes methane ($CH_4$), propane ($C_3H_8$), nitrogen ($N_2$), and carbon dioxide ($CO_2$) at an unknown volume ratio is introduced into the chamber 101. The first temperature measurement element 62 of the microchip 8 shown in Figs. 1 and 2 is supplied with a weak voltage not enough to generate self-heating. Next, the heater element 61 is sequentially supplied with the voltages $V_{L1}$, $V_{L2}$, and $V_{L3}$ from the driving circuit 303 shown in Fig. 6. When the voltages $V_{L1}$, $V_{L2}$, and $V_{L3}$ are applied, the heater element 61 coming into contact with the measurement target mixed gas generates heat at a temperature $T_{H1}$ near, for example, 100°C, a temperature $T_{H2}$ near 150°C, and a temperature $T_{H3}$ near 200°C, respectively.

A measurement module 331 for calorific value calculation shown in Fig. 11 measures the value of the resistance $R_I$ of the first temperature measurement element 62 coming into contact with the measurement target mixed gas, the value of the resistance $R_H(V_{L1})$ of the heater element 61, coming into contact with the measurement target mixed gas, to which the voltage $V_{L1}$ is applied, the value of the resistance $R_H(V_{L2})$ of the heater element 61 to which the voltage $V_{L2}$ is applied, and the value of the resistance $R_H(V_{L3})$ of the heater element 61 to which the voltage $V_{L3}$ is applied, and saves the measured values in the electric signal storage device 401.

The calorific value calculation module 305 respectively substitutes the measured values into the independent variables which are the resistance $R_I$ of the first temperature measurement element 62 and the resistances $R_H(V_{L1})$, $R_H(V_{L2})$, and $R_H(V_{L3})$ of the heater element 61 in the calorific value calculation formula to calculate the value of the calorific value Q of the measurement target mixed gas. A calorific value storage device 403 is connected to the CPU 300. The calorific value storage device 403 saves the value of the calorific value Q of the measurement target mixed gas, which is calculated by the calorific value calculation module 305. Since the other constituent requirements for the calorific value measurement system 21 according to the fourth embodiment are the same as those for the calorific value calculation formula creation system 20 according to the first embodiment described in Fig. 6, a description thereof will be omitted.

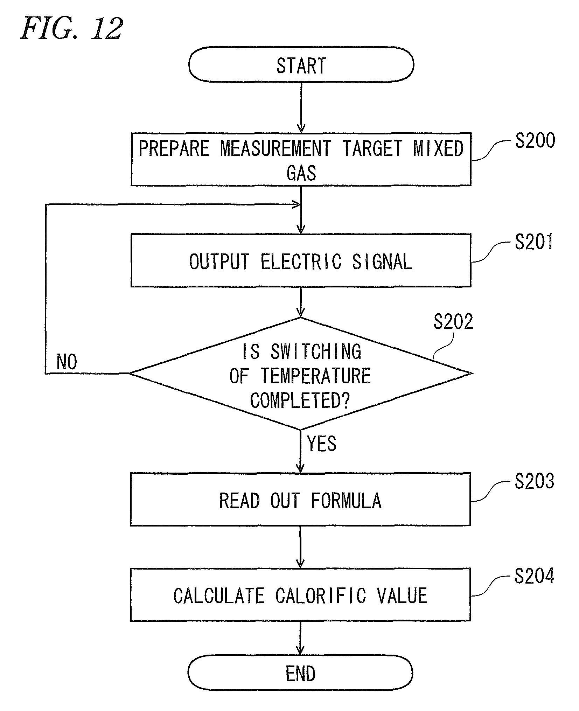

Next, a method of measuring a calorific value according to the fourth embodiment will be described using a flowchart shown in Fig. 12.

(a) In Step S200, the heat-retaining element 64 shown in Figs. 1 and 2 is allowed to generate heat to constantly maintain the temperature of the substrate 60 to, for example, 60°C. Next, a measurement target mixed gas is introduced into the chamber 101 shown in Fig. 11. In Step S201, the measurement module 331 measures the value of the resistance $R_I$ of the first temperature measurement element 62 coming into contact with the measurement target mixed gas and saves the measured value in the electric signal storage device 401. After that, the driving circuit 303 applies the voltage $V_{L1}$ to the heater element 61 shown in Figs. 1 and 2. The measurement module 331 shown in Fig. 11 saves the value of the resistance $R_H(V_{L1})$ of the heater element 61, coming into contact with the measurement target mixed gas, to which the voltage $V_{L1}$ is applied in the electric signal storage device 401.

(b) In Step S202, the driving circuit 303 shown in Fig. 11 determines whether or not the switching of the voltage $V_L$ which is applied to the heater element 61 shown in Figs. 1 and 2 is completed. When the switching to the voltages $V_{L2}$ and $V_{L3}$ is not completed, the process returns to Step S201 and the driving circuit 303 applies the voltage $V_{L2}$ to the heater element 61 shown in Figs. 1 and 2. The measurement module 331 shown in Fig. 11 saves the value of the resistance $R_H(V_{L2})$ of the heater element 61, coming into contact with the measurement target mixed gas, to which the voltage $V_{L2}$ is applied in the electric signal storage device 401.

(c) The driving circuit 303 determines again whether or not the switching of the voltage $V_L$ which is applied to the heater element 61 shown in Figs. 1 and 2 is completed in Step S202. When the switching to the voltage $V_{L3}$ is not completed, the process returns to Step S201 and the driving circuit 303 shown in Fig. 11 applies the voltage $V_{L3}$ to the heater element 61 shown in Figs. 1 and 2. The measurement module 331 shown in Fig. 11 saves the value of the resistance $R_H(V_{L3})$ of the heater element 61, coming into contact with the measurement target mixed gas, to which the voltage $V_{L3}$ is applied in the electric signal storage device 401.

(d) When the switching to the voltage $V_L$ which is applied to the heater element 61 is completed, the process advances to Step S203 from Step S202. In Step S203, the calorific value calculation module 305 shown in Fig. 11 reads out a calorific value calculation formula having the resistance $R_I$ of the first temperature measurement element 62 and the resistances $R_H(V_{L1})$, $R_H(V_{L2})$, and $R_H(V_{L3})$ of the heater element 61 as independent variables and the calorific value Q as a dependent variable from the calorific value calculation formula storage device 402. In addition, the calorific value calculation module 305 reads out the measured value of the resistance $R_I$ of the first temperature measurement element 62 coming into contact with the measurement target mixed gas and the measured values of the resistances $R_H(V_{L1})$, $R_H(V_{L2})$, and $R_H(V_{L3})$ of the heater element 61 coming into contact with the measurement target mixed gas from the electric signal storage device 401.

(e) In Step S204, the calorific value calculation module 305 respectively substitutes the measured values into the independent variables which are the resistance $R_I$ and the resistances $R_H(V_{L1})$, $R_H(V_{L2})$, and $R_H(V_{L3})$ in the calorific value calculation formula to calculate the value of the calorific value Q of the measurement target mixed gas. After that, the calorific value calculation module 305 saves the calculated value of the calorific value Q in the calorific value storage device 403 and the method of measuring a calorific value according to the fourth embodiment ends.

According to the above-described method of calculating a calorific value of the fourth embodiment, it is possible to measure the value of the calorific value Q of the measurement target mixed gas from the value of the resistance $R_I$ of the first temperature measurement element 62 coming into contact with the measurement target mixed gas and the values of $R_H(V_{L1})$, $R_H(V_{L2})$, and $R_H(V_{L3})$ of the heater element 61 coming into contact with the measurement target mixed gas without using an expensive gas chromatography device or sound speed sensor.

The percentage of carbon hydride in the natural gases varies depending on the gas field. In addition, the natural gases include, for example, nitrogen ($N_2$) or carbon dioxide ($CO_2$) in addition to carbon hydride. Therefore, the volume ratio of gas components in the natural gases varies depending on the gas field. Even when the kind of gas components is known, in many cases, the calorific value Q of the natural gas is unknown. In addition, the natural gases with different calorific values Q may be produced from the same gas field and the calorific value Q of the natural gases varies depending on the production period.

In the related art, when the a price for use of the natural gas is charged, a charging method based on the volume of the natural gas used, not the calorific value Q of the natural gas used has been used. However, it is unfair to charge for the volume of the natural gas used since the calorific value Q of the natural gas varies depending on the gas field of the natural gas. In contrast, when the method of calculating the calorific value according to the fourth embodiment is used, it is possible to easily calculate the calorific value Q of a mixed gas, such as a natural gas whose calorific value Q is unknown, since the kind of gas components is known, but the volume ratio of the gas components is unknown. Therefore, it is possible to fairly charge for the use of the mixed gas.

In the glass product manufacturing industry, when glass is heated and processed, it is preferable to supply a natural gas with a constant calorific value Q in order to maintain processing accuracy to be constant. In order to maintain processing accuracy, a technique has been examined which accurately checks the calorific values Q of the natural gases produced from a plurality of gas fields, adjusts the calorific values Q of all of the natural gases to be equal to each other, and supplies the natural gas for a process of heating and processing glass. In contrast, when the method of calculating the calorific value according to the fourth embodiment is used, it is possible to accurately check the calorific values Q of the natural gases produced from a plurality of gas fields. Therefore, it is possible to maintain the heating and processing accuracy of glass to be constant.

According to the method of calculating the calorific value of the fourth embodiment, it is possible to easily know the accurate calorific value Q of a mixed gas, such as a natural gas, and thus appropriately set the amount of air required to burn the mixed gas. Therefore, it is possible to reduce the amount of carbon dioxide ($CO_2$) emitted.

(Fifth Embodiment)

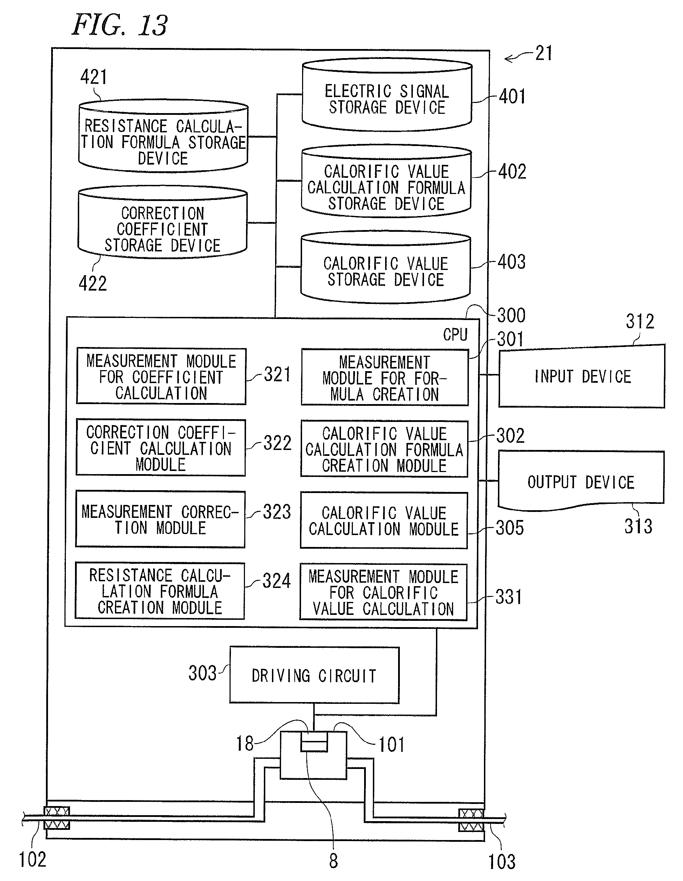

As shown in Fig. 13, a calorific value measurement system 21 according to a fifth embodiment further includes a resistance calculation formula creation module 324, a resistance calculation formula storage device 421, a measurement module 321 for coefficient calculation, a correction coefficient calculation module 322, a correction coefficient storage device 422, and a measurement correction module 323 in addition to the constituent elements in the calorific value measurement system 21 according to the fourth embodiment shown in Fig. 11. Since the resistance calculation formula creation module 324, the resistance calculation formula storage device 421, the measurement module 321, the correction coefficient calculation module 322, and the correction coefficient storage device 422 are the same as in the second embodiment, a description thereof will be omitted herein.

The measurement correction module 323 corrects the measured values indicating the resistances $R_H(V_{L1})$, $R_H(V_{L2})$, and $R_H(V_{L3})$ of the heater element 61 coming into contact with the measurement target mixed gas, which are measured by the measurement module 331 using the correction coefficients $cf(V_{L1})$, $cf(V_{L2})$, and $cf(V_{L3})$. In the fifth embodiment, the calorific value calculation module 305 substitutes the measured value of the resistance $R_I$ of the first temperature measurement element 62 coming into contact with the measurement target mixed gas into the independent variable which is the resistance $R_I$ of the first temperature measurement element 62 in the calorific value calculation formula. In addition, the calorific value calculation module 305 substitutes the corrected measured values $R_{H\_CR}(V_{L1})$, $R_{H\_CR}(V_{L2})$, and $R_{H\_CR}(V_{L3})$ of the resistances of the heater element 61 coming into contact with the measurement target mixed gas into the independent variables which are the resistances $R_H(V_{L1})$, $R_H(V_{L2})$, and $R_H(V_{L3})$ of the heater element 61 in the calorific value calculation formula to calculate the value of the calorific value Q of the measurement target mixed gas.

According to the calorific value measurement system 21 in the first embodiment, it is possible to calculate the value of the calorific value Q of the measurement target mixed gas with higher accuracy.

(Sixth Embodiment)

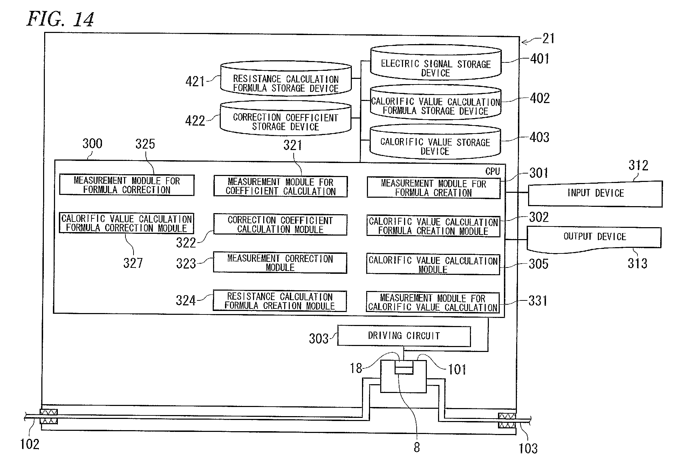

As shown in Fig. 14, a calorific value measurement system 21 according to a sixth embodiment further includes a measurement module 325 for formula correction and a calorific value calculation formula correction module 327 in addition to the constituent elements in the calorific value measurement system 21 according to the fifth embodiment shown in Fig. 13. Since the measurement module 325 and the calorific value calculation formula correction module 327 are the same as those in the third embodiment, a description thereof will be omitted herein. According to the calorific value measurement system 21 of the sixth embodiment, it is possible to calculate the value of the calorific value Q of the measurement target mixed gas with higher accuracy by correcting the calorific value calculation formula.

(Example 1)

First, twenty-three kinds of sample mixed gases having known calorific values Q were prepared. Each of the twenty-three kinds of sample mixed gases included as gas components any one or all of methane ($CH_4$), ethane ($C_2H_6$), propane ($C_3H_8$), butane ($C_4H_{10}$), nitrogen ($N_2$), and carbon dioxide ($CO_2$). For example, a sample mixed gas included 90 vol% of methane, 3 vol% of ethane, 1 vol% of propane, 1 vol% of butane, 4 vol% of nitrogen, and 1 vol% of carbon dioxide. A sample mixed gas included 85 vol% of methane, 10 vol% of ethane, 3 vol% of propane, and 2 vol% of butane, but did not include nitrogen and carbon dioxide. A sample mixed gas included 85 vol% of methane, 8 vol% of ethane, 2 vol% of propane, 1 vol% of butane, 2 vol% of nitrogen, and 2 vol% of carbon dioxide.

Next, a plurality of measured values of the resistance $R_I$ of the first temperature measurement element 62 shown in Fig. 6 and a plurality of measured values of the resistances $R_H(V_{L1})$, $R_H(V_{L2})$, and $R_H(V_{L3})$ of the heater element 61 were obtained using the twenty-three kinds of sample mixed gases. After that, based on the values of the known calorific values Q of the twenty-three kinds of sample mixed gases, the plurality of measured values of the resistance $R_I$ of the first temperature measurement element 62 and the plurality of measured values of the resistances $R_H(V_{L1})$, $R_H(V_{L2})$, and $R_H(V_{L3})$ of the heater element 61, a linear equation, a quadratic equation, and a cubic equation, that were for calculating a calorific value Q and had the resistance $R_I$ of the first temperature measurement element 62 and the resistances $R_H(V_{L1})$, $R_H(V_{L2})$, and $R_H(V_{L3})$ of the heater element 61 as independent variables and the calorific value Q as a dependent variable, were created by support vector regression.

When creating the linear equation for calculating the calorific value Q, it can be appropriately determined that there are approximately three to five calibration points. The created linear equation is represented by the following Expression (42). When the calorific values Q of the twenty-three kinds of sample mixed gases were calculated by Expression (42) and compared with true calorific values, the maximum error was 2.1 %.

$$Q = 40.1 + 23.8 \times R_H(V_{L1}) + 6.07 \times R_H(V_{L2}) - 22.8 \times R_H(V_{L3}) - 11.4 \times R_I$$

$$\dots(42)$$

When creating the quadratic equation for calculating the calorific value Q, it can be appropriately determined that there are approximately eight to nine calibration points. When the calorific values Q of the twenty-three kinds of sample mixed gases were calculated by the created quadratic equation and compared with true calorific values, the maximum error was 1.2 to 1.4%.

When creating the cubic equation for calculating the calorific value Q, it can be appropriately determined that there are approximately ten to fourteen calibration points. When the calorific values Q of the twenty-three kinds of sample mixed gases were calculated by the created cubic equation and compared with true calorific values, the maximum error was less than 1.2%.

(Example 2)

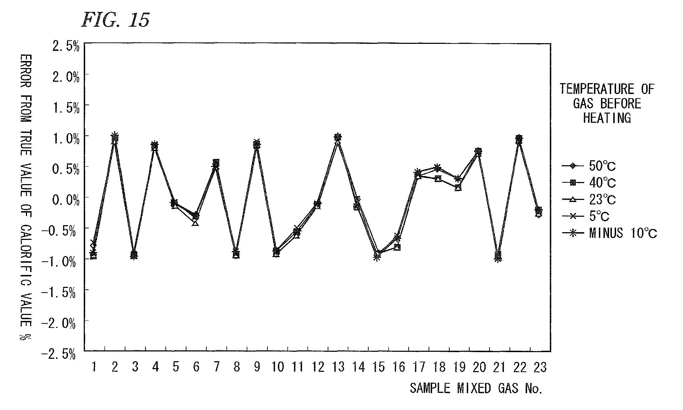

As in the case of the sample mixed gases used in Example 1, twenty-three kinds of sample mixed gases having known calorific values Q were prepared. Here, the temperature of the sample mixed gas before heating by the heater element 61 was set to -10°C, 5°C, 23°C, 40°C, and 50°C. Next, a cubic equation, that was for calculating the calorific value Q and had the resistance $R_I$ of the first temperature measurement element 62 and the resistances $R_H(V_{L1})$, $R_H(V_{L2})$, and $R_H(V_{L3})$ of the heater element 61 as independent variables and the calorific value Q as a dependent variable, was created by support vector regression. Accordingly, as shown in Fig. 15, no fluctuation occurred in the error of the calculated calorific value Q regardless of the temperature of the sample mixed gas before heating by the heater element 61.

**Claims**

1. A resistance measurement system comprising:

   a resistance calculation formula storage device (421) which is arranged to store a resistance calculation formula, wherein the resistance calculation formula is represented by:

   a first temperature detected by a temperature measurement element (62) coming into contact with a calibration gas, the first temperature being set as an independent variable in the resistance calculation formula; and
   a first resistance of a heater element (61) coming into contact with the calibration gas, the resistance being set as a dependent variable in the resistance calculation formula,

   a first measurement module (321) which is arranged to obtain a measured value of the first temperature and a measured value of the first resistance;
   a correction coefficient calculation module (322) which is arranged to:

   substitute the measured value of the first temperature into the first temperature that is set as the independent variable in the resistance calculation formula so as to obtain a calculated value of the first resistance; and calculate a correction coefficient that represents a ratio between the measured value of the first resistance and the calculated value of the first resistance; and

   a measurement correction module (323) which corrects a measured value of the first resistance, based on the correction coefficient.

2. A calorific value calculation formula creation system (20) comprising:

   a second measurement module (301) which is arranged to obtain:

   a measured value of a second resistance of the temperature measurement element coming into contact with a mixed gas including a plurality of gases; and
   a measured value of the first resistance of the heater element coming into contact with the mixed gas including the plurality of gases,

   the resistance measurement system according to claim **1,** wherein the measurement correction module (323) is arranged to correct the measured value of the first resistance of the heater element coming into contact with the mixed gas, based on the correction coefficient; and
   a calorific value calculation formula creation module (302) which is arranged to create a calorific value calculation formula, based on: calorific values of the plurality of gases included in the mixed gas; the measured value of the second resistance of the temperature measurement element coming into contact with the mixed gas; and the corrected measured value of the first resistance of the heater element,

   wherein the calorific value calculation formula is represented by:

   the second resistance of the temperature measurement element that is set as an independent variable in the calorific value calculation formula;
   the first resistance of the heater element that is set as an independent variable in the calorific value calculation formula; and
   a calorific value of the mixed gas that is set as a dependent variable in the calorific value calculation formula.

3. The system according to Claim **2,** further comprising:

   a third measurement module (325) which is arranged to obtain:

   a measured value of the second resistance of the temperature measurement element coming into contact with the calibration gas; and

the measured value of the first resistance of the heater element coming into contact with the calibration gas, a calorific value calculation module (305) which is arranged to substitute the measured value of the second resistance and the measured value of the first resistance which is corrected by the correction coefficient, into the second resistance of the temperature measurement element and the first resistance of the heater element, both of which are set as independent variables in the calorific value calculation formula, so as to obtain a calculated value of a calorific value of the calibration gas; and
a calorific value calculation formula correction module (327) which corrects the calorific value calculation formula such that the calculated value of the calorific value of the calibration gas is equal to the calorific value of the calibration gas which is obtained in advance.

4. The system according to claim **2** or **3,**
wherein a plurality of voltages is applied to the heater element, and
the number of the voltages is greater than or equal to a number that is one less than the number of the plurality of gases included in the mixed gas.

5. The system according to any one of claims **2** to **4,**
wherein the calorific value calculation formula creation module is arranged to create the calorific value calculation formula using support vector regression.

6. A calorific value measurement system comprising:

a second measurement module (331) which is arranged to obtain:

a measured value of a second resistance of the temperature measurement element (62) coming into contact with a mixed gas including a plurality of gases; and
a measured value of the first resistance of the heater element coming into contact with the mixed gas;

the resistance measurement system according to claim **1,** wherein the measurement correction module (323) is arranged to correct the measured value of the first resistance of the heater element coming into contact with the mixed gas, based on the correction coefficient;
a calorific value calculation formula storage device (402) which is arranged to store a calorific value calculation formula, wherein the calorific value calculation formula is represented by:

the second resistance of the temperature measurement element that is set as an independent variable in the calorific value calculation formula;
the first resistance of the heater element that is set as an independent variable in the calorific value calculation formula; and
a calorific value that is set as a dependent variable in the calorific value calculation formula; and

a calorific value calculation module (305) which is arranged to substitute the measured value of the second resistance of the temperature measurement element coming into contact with the mixed gas and the corrected measured value of the first resistance of the heater element coming into contact with the mixed gas, into the second resistance of the temperature measurement element and the first resistance of the heater element, both of which are set as independent variables in the calorific value calculation formula, so as to calculate a calorific value of the mixed gas.

7. The system according to Claim **6,** further comprising:

a third measurement module (325) which is arranged to obtain:

a measured value of the second resistance of the temperature measurement element coming into contact with the calibration gas; and

the measured value of the first resistance of the heater element coming into contact with the calibration gas; and
a calorific value calculation formula correction module (327) which is arranged to correct the calorific value calculation formula such that the calculated calorific value of the calibration gas is equal to the calorific value of the calibration gas which is obtained in advance,

wherein the calorific value calculation module is arranged to substitute the measured value of the second resistance and the measured value of the first resistance which is corrected by the correction coefficient, into the second resistance of the temperature measurement element and the first resistance of the heater element, both of which are set as independent variables in the calorific value calculation formula, so as to obtain a calculated value of the calorific value of the calibration gas.

8. The system according to claim **6** or **7,**
   wherein a plurality of voltages is applied to the heater element, and
   the number of the voltages is greater than or equal to a number that is one less than the number of the plurality of gases included in the mixed gas.

9. A method of measuring a resistance, the method comprising:

   (a) preparing a resistance calculation formula, wherein the resistance calculation formula is represented by:

   a first temperature detected by a temperature measurement element (62) coming into contact with a calibration gas, the first temperature being set as an independent variable in the resistance calculation formula; and
   a first resistance of a heater element (61) coming into contact with the calibration gas, the resistance being set as a dependent variable in the resistance calculation formula,

   (b) obtaining a measured value of the first temperature and a measured value of the first resistance;
   (c) substituting the measured value of the first temperature into the first temperature that is set as the independent variable in the resistance calculation formula so as to obtain a calculated value of the first resistance; and
   (d) calculating a correction coefficient that represents a ratio between the measured value of the first resistance and the calculated value of the first resistance; and
   (e) correcting a measured value of the first resistance, based on the correction coefficient.

10. A method of creating a calorific value calculation formula, the method comprising determining a correction coefficient according to claim 9, and comprising the following steps :

    (a) obtaining a measured value of a second resistance of a temperature measurement element coming into contact with a mixed gas including a plurality of gases;
    (b) obtaining a measured value of a first resistance of a heater element coming into contact with the mixed gas including the plurality of gases;
    (c) correcting the measured value of the first resistance of the heater element coming into contact with the mixed gas, based on said correction coefficient; and
    (d) creating a calorific value calculation formula, based on: calorific values of the plurality of gases included in the mixed gas; the measured value of the second resistance of the temperature measurement element coming into contact with the mixed gas; and the corrected measured value of the first resistance of the heater element, wherein the calorific value calculation formula is represented by:

    the second resistance of the temperature measurement element that is set as an independent variable in the calorific value calculation formula;
    the first resistance of the heater element that is set as an independent variable in the calorific value calculation formula; and
    a calorific value of the mixed gas that is set as a dependent variable in the calorific value calculation formula.

11. The method according to Claim **10,** further comprising:

    (e) obtaining a measured value of the second resistance of the temperature measurement element coming into contact with a calibration gas, and the measured value of the first resistance of the heater element coming into contact with the calibration gas,
    (f) substituting the measured value of the second resistance and the measured value of the first resistance which is corrected by the correction coefficient, into the second resistance of the temperature measurement element and the first resistance of the heater element, both of which are set as independent variables in the calorific value calculation formula, so as to obtain a calculated value of a calorific value of the calibration gas; and
(g) correcting the calorific value calculation formula such that the calculated value of the calorific value of the calibration gas is equal to the calorific value of the calibration gas which is obtained in advance.

**12.** The method according to claim **10** or **11,**
wherein a plurality of voltages is applied to the heater element, and
the number of the voltages is greater than or equal to a number that is one less than the number of the plurality of gases included in the mixed gas.

**13.** The method according to any one of claims **10** to **12,**
wherein step (d) comprises: creating the calorific value calculation formula using support vector regression.

**14.** A method of measuring a calorific value, the method comprising:

(a) obtaining a measured value of a second resistance of a temperature measurement element (62) coming into contact with a mixed gas including a plurality of gases, and a measured value of a first resistance of a heater element coming into contact with the mixed gas;
(b) correcting the measured value of the first resistance of the heater element coming into contact with the mixed gas, based on a correction coefficient by the method according to Claim **9;**
(c) preparing a calorific value calculation formula, wherein the calorific value calculation formula is represented by:

the second resistance of the temperature measurement element that is set as an independent variable in the calorific value calculation formula;
the first resistance of the heater element that is set as an independent variable in the calorific value calculation formula; and
a calorific value that is set as a dependent variable in the calorific value calculation formula,

(d) substituting the measured value of the second resistance of the temperature measurement element coming into contact with the mixed gas and the corrected measured value of the first resistance of the heater element coming into contact with the mixed gas, into the second resistance of the temperature measurement element and the first resistance of the heater element, both of which are set as independent variables in the calorific value calculation formula, so as to calculate a calorific value of the mixed gas.

**15.** The method according to Claim **14,** further comprising:

(e) obtaining a measured value of the second resistance of the temperature measurement element coming into contact with a calibration gas, and the measured value of the first resistance of the heater element coming into contact with the calibration gas;
(f) substituting the measured value of the second resistance and the measured value of the first resistance which is corrected by the correction coefficient, into the second resistance of the temperature measurement element and the first resistance of the heater element, both of which are set as independent variables in the calorific value calculation formula, so as to obtain a calculated value of the calorific value of the calibration gas; and
(g) correcting the calorific value calculation formula such that the calculated calorific value of the calibration gas is equal to the calorific value of the calibration gas which is obtained in advance.

**16.** The method according to claim **14** to **15,**
wherein a plurality of voltages is applied to the heater element, and
the number of the voltages is greater than or equal to a number that is one less than the number of the plurality of gases included in the mixed gas.

**Patentansprüche**

**1.** Widerstandsmesssystem, umfassend:

eine Widerstandsberechnungsformel-Speichervorrichtung (421), die ausgelegt ist,
eine Widerstandsberechnungsformel zu speichern, wobei die

Widerstandsberechnungsformel repräsentiert wird durch:

eine erste Temperatur, die von einem Temperaturmesselement (62) detektiert wird, das mit einem Kalibriergas in Kontakt gelangt, wobei die erste Temperatur als unabhängige Variable in die Widerstandsberechnungsformel eingesetzt wird, und

einen ersten Widerstand eines Heizerelements (61), das mit dem Kalibriergas in Kontakt gelangt, wobei der Widerstand als abhängige Variable in die Widerstandsberechnungsformel eingesetzt wird;

ein erstes Messmodul (321), das ausgelegt ist, einen gemessenen Wert der ersten Temperatur und einen gemessenen Wert des ersten Widerstands zu erhalten; ein Korrekturkoeffizient-Berechnungsmodul (322), das ausgelegt ist:

die erste Temperatur, die als unabhängige Variable in die Widerstandsberechnungsformel eingesetzt ist, durch den gemessenen Wert der ersten Temperatur zu substituieren, um so einen berechneten Wert des ersten Widerstands zu erhalten; und

einen Korrekturkoeffizienten zu berechnen, der ein Verhältnis zwischen dem gemessenen Wert des ersten Widerstands und dem berechneten Wert des ersten Widerstands repräsentiert; und

ein Messkorrekturmodul (323), das einen gemessenen Wert des ersten Widerstands auf der Basis des Korrekturkoeffizienten korrigiert.

2. Heizwertberechnungsformel-Erstellungssystem (20), umfassend:

ein zweites Messmodul (301), das ausgelegt ist zu erhalten:

einen gemessenen Wert eines zweiten Widerstands des Temperaturmesselements, welches mit einem gemischten Gas in Kontakt gelangt, das mehrere Gase enthält, und

einen gemessenen Wert des ersten Widerstands des Heizerelements, welches mit dem gemischten Gas in Kontakt gelangt, das die mehreren Gase enthält;

das Widerstandsmesssystem nach Anspruch 1, wobei das Messkorrekturmodul (323) ausgelegt ist, den gemessenen Wert des ersten Widerstands des Heizerelements, das mit dem gemischten Gas in Kontakt gelangt, auf der Basis des Korrekturkoeffizienten zu korrigieren; und

ein Heizwertberechnungsformel-Erstellungsmodul (302), das ausgelegt ist, eine Heizwertberechnungsformel zu erstellen, auf der Basis von: Heizwerten der mehreren Gase, die in dem gemischten Gas enthalten sind; dem gemessenen Wert des zweiten Widerstands des Temperaturmesselements, das mit dem gemischten Gas in Kontakt gelangt; und dem korrigierten gemessenen Wert des ersten Widerstands des Heizerelements,

wobei die Heizwertberechnungsformel repräsentiert wird durch:

den zweiten Widerstand des Temperaturmesselements, der als unabhängige Variable in die Heizwertberechnungsformel eingesetzt wird;

den ersten Widerstand des Heizerelements, der als unabhängige Variable in die Heizwertberechnungsformel eingesetzt wird; und

einen Heizwert des gemischten Gases, der als abhängige Variable in die Heizwertberechnungsformel eingesetzt wird.

3. System nach Anspruch 2, ferner umfassend:

ein drittes Messmodul (325), das ausgelegt ist zu erhalten:

einen gemessenen Wert des zweiten Widerstands des Temperaturmesselements, das mit dem Kalibriergas in Kontakt gelangt, und

den gemessenen Wert des ersten Widerstands des Heizerelements, das mit dem Kalibriergas in Kontakt gelangt;

ein Heizwertberechnungsmodul (305), das ausgelegt ist, den zweiten Widerstand des Temperaturmesselements und den ersten Widerstand des Heizerelements, von denen beide als unabhängige Variablen in die Heizwertberechnungsformel eingesetzt sind, durch den gemessenen Wert des zweiten Widerstands und den gemessenen Wert des ersten Widerstands, der durch den Korrekturkoeffizienten korrigiert ist, zu substituieren, um so einen berechneten Wert eines Heizwerts des Kalibriergases zu erhalten; und ein Heizwertberechnungsformel-Korrekturmodul (327), das die Heizwertberechnungsformel korrigiert, so dass der berechnete Wert des Heizwerts des Kalibriergases gleich dem Heizwert des Kalibriergases ist, der im Voraus erhalten wird.

4. System nach Anspruch 2 oder 3,
wobei mehrere Spannungen an das Heizerelement angelegt werden, und die Anzahl der Spannungen größer oder gleich einer Anzahl ist, die um Eins kleiner ist als die Anzahl der mehreren Gase, die in dem gemischten Gas enthalten sind.

5. System nach einem der Ansprüche 2 bis 4,
wobei das Heizwertberechnungsformel-Erstellungsmodul ausgelegt ist, die Heizwertberechnungsformel unter Verwendung einer Support-Vector-Regression zu erstellen.

6. Heizwertmesssystem, umfassend:

ein zweites Messmodul (331), das ausgelegt ist zu erhalten:

einen gemessenen Wert des zweiten Widerstands des Temperaturmesselements (62), welches mit einem gemischten Gas in Kontakt gelangt, das mehrere Gase enthält, und
einen gemessenen Wert des ersten Widerstands des Heizerelements, das mit dem gemischten Gas in Kontakt gelangt;
das Widerstandsmesssystem nach Anspruch 1, wobei das Messkorrekturmodul (323) ausgelegt ist, den gemessenen Wert des ersten Widerstands des Heizerelements, das mit dem gemischten Gas in Kontakt gelangt, auf der Basis des Korrekturkoeffizienten zu korrigieren;
eine Heizwertberechnungsformel-Speichervorrichtung (402), die ausgelegt ist,
eine Heizwertberechnungsformel zu speichern, wobei die

Heizwertberechnungsformel repräsentiert wird durch:

den zweiten Widerstand des Temperaturmesselements, der als unabhängige Variable in die Heizwertberechnungsformel eingesetzt wird;
den ersten Widerstand des Heizerelements, der als unabhängige Variable in die Heizwertberechnungsformel eingesetzt wird; und
einen Heizwert, der als abhängige Variable in die Heizwertberechnungsformel eingesetzt wird; und
ein Heizwertberechnungsmodul (305), das ausgelegt ist, den zweiten Widerstand des Temperaturmesselements und den ersten Widerstand des Heizerelements, von denen beide als unabhängige Variablen in die Heizwertberechnungsformel eingesetzt sind, durch den gemessenen Wert des zweiten Widerstands des Temperaturmesselements, das mit dem gemischten Gas in Kontakt gelangt, und den korrigierten gemessenen Wert des ersten Widerstands des Heizerelements, das mit dem gemischten Gas in Kontakt gelangt, zu substituieren, um so einen Heizwert des gemischten Gases zu berechnen.

7. System nach Anspruch 6, ferner umfassend:

ein drittes Messmodul (325), das ausgelegt ist zu erhalten:

einen gemessenen Wert des zweiten Widerstands des Temperaturmesselements, das mit dem Kalibriergas in Kontakt gelangt, und
den gemessenen Wert des ersten Widerstands des Heizerelements, das mit dem Kalibriergas in Kontakt gelangt; und

ein Heizwertberechnungsformel-Korrekturmodul (327), das ausgelegt ist, die Heizwertberechnungsformel zu korrigieren, so dass der berechnete Heizwert des Kalibriergases gleich dem Heizwert des Kalibriergases ist, der im Voraus erhalten wird,

wobei das Heizwertberechnungsmodul ausgelegt ist, den zweiten Widerstand des Temperaturmesselements und den ersten Widerstand des Heizerelements, von denen beide als unabhängige Variablen in die Heizwertberechnungsformel eingesetzt sind, durch den gemessenen Wert des zweiten Widerstands und den gemessenen Wert des ersten Widerstands, der durch den Korrekturkoeffizienten korrigiert ist, zu substituieren, um so einen berechneten Wert des Heizwerts des Kalibriergases zu erhalten.

8. System nach Anspruch 6 oder 7,
wobei mehrere Spannungen an das Heizerelement angelegt werden, und
die Anzahl der Spannungen größer oder gleich einer Anzahl ist, die um Eins kleiner ist als die Anzahl der mehreren Gase, die in dem gemischten Gas enthalten sind.

9. Verfahren zum Messen eines Widerstands, wobei das Verfahren umfasst:

(a) Erstellen einer Widerstandsberechnungsformel, wobei die Widerstandsberechnungsformel repräsentiert wird durch:

eine erste Temperatur, die von einem Temperaturmesselement (62) detektiert wird, das mit einem Kalibriergas in Kontakt gelangt, wobei die erste Temperatur als unabhängige Variable in die Widerstandsberechnungsformel eingesetzt wird, und
einen ersten Widerstand eines Heizerelements (61), das mit dem Kalibriergas in Kontakt gelangt, wobei der Widerstand als abhängige Variable in die Widerstandsberechnungsformel eingesetzt wird;

(b) Erhalten eines gemessenen Werts der ersten Temperatur und einen gemessenen Wert des ersten Widerstands;
(c) Substituieren der ersten Temperatur, die als unabhängige Variable in die Widerstandsberechnungsformel eingesetzt ist, durch den gemessenen Wert der ersten Temperatur, um so einen berechneten Wert des ersten Widerstands zu erhalten; und
(d) Berechnen eines Korrekturkoeffizienten, der ein Verhältnis zwischen dem gemessenen Wert des ersten Widerstands und dem berechneten Wert des ersten Widerstands repräsentiert; und
(e) Korrigieren eines gemessenen Werts des ersten Widerstands auf der Basis des Korrekturkoeffizienten.

10. Verfahren zum Erstellen einer Heizwertberechnungsformel, wobei das Verfahren das Bestimmen eines Korrekturkoeffizienten nach Anspruch 9 umfasst und die folgenden Schritte umfasst:

(a) Erhalten eines gemessenen Werts eines zweiten Widerstands eines Temperaturmesselements, welches mit einem gemischten Gas in Kontakt gelangt, das mehrere Gase enthält; und
(b) Erhalten eines gemessenen Werts eines ersten Widerstands eines Heizerelements, welches mit dem gemischten Gas in Kontakt gelangt, das die mehreren Gase enthält,
(c) Korrigieren des gemessenen Werts des ersten Widerstands des Heizerelements, das mit dem gemischten Gas in Kontakt gelangt, auf der Basis des Korrekturkoeffizienten; und
(d) Erstellen einer Heizwertberechnungsformel, auf der Basis von: Heizwerten der mehreren Gase, die in dem gemischten Gas enthalten sind; dem gemessenen Wert des zweiten Widerstands des Temperaturmesselements, das mit dem gemischten Gas in Kontakt gelangt; und dem korrigierten gemessenen Wert des ersten Widerstands des Heizerelements,

wobei die Heizwertberechnungsformel repräsentiert wird durch:

den zweiten Widerstand des Temperaturmesselements, der als unabhängige Variable in die Heizwertberechnungsformel eingesetzt wird;
den ersten Widerstand des Heizerelements, der als unabhängige Variable in die Heizwertberechnungsformel eingesetzt wird; und
einen Heizwert des gemischten Gases, der als abhängige Variable in die Heizwertberechnungsformel eingesetzt wird.

11. Verfahren nach Anspruch 10, ferner umfassend:

(e) Erhalten eines gemessenen Werts des zweiten Widerstands des Temperaturmesselements, das mit einem Kalibriergas in Kontakt gelangt, und des gemessenen Werts des ersten Widerstands des Heizerelements, das mit dem Kalibriergas in Kontakt gelangt;
(f) Substituieren des zweiten Widerstands des Temperaturmesselements und des ersten Widerstands des Heizerelements, von denen beide als unabhängige Variablen in die Heizwertberechnungsformel eingesetzt sind, durch den gemessenen Wert des zweiten Widerstands und den gemessenen Wert des ersten Widerstands, der durch den Korrekturkoeffizienten korrigiert ist, um so einen berechneten Wert eines Heizwerts des Kalibriergases zu erhalten; und

(g) Korrigieren der Heizwertberechnungsformel, so dass der berechnete Wert des Heizwerts des Kalibriergases gleich dem Heizwert des Kalibriergases ist, der im Voraus erhalten wird.

12. Verfahren nach Anspruch 10 oder 11,
wobei mehrere Spannungen an das Heizerelement angelegt werden, und
die Anzahl der Spannungen größer oder gleich einer Anzahl ist, die um Eins kleiner ist als die Anzahl der mehreren Gase, die in dem gemischten Gas enthalten sind.

13. Verfahren nach einem der Ansprüche 10 bis 12,
wobei Schritt (d) umfasst: Erstellen der Heizwertberechnungsformel unter Verwendung einer Support-Vector-Regression.

14. Verfahren zum Messen eines Heizwerts, wobei das Verfahren umfasst:

(a) Erhalten eines gemessenen Werts eines zweiten Widerstands eines Temperaturmesselements (62), welches mit einem gemischten Gas in Kontakt gelangt, das mehrere Gase enthält, und eines gemessenen Werts eines ersten Widerstands eines Heizerelements, das mit dem gemischten Gas in Kontakt gelangt;
(b) Korrigieren des gemessenen Werts des ersten Widerstands des Heizerelements, das mit dem gemischten Gas in Kontakt gelangt, auf der Basis eines Korrekturkoeffizienten durch das Verfahren nach Anspruch 9;
(c) Erstellen einer Heizwertberechnungsformel, wobei die Heizwertberechnungsformel repräsentiert wird durch:

den zweiten Widerstand des Temperaturmesselements, der als unabhängige Variable in die Heizwertberechnungsformel eingesetzt wird;
den ersten Widerstand des Heizerelements, der als unabhängige Variable in die Heizwertberechnungsformel eingesetzt wird; und
einen Heizwert, der als abhängige Variable in die Heizwertberechnungsformel eingesetzt wird; und

(d) Substituieren des zweiten Widerstands des Temperaturmesselements und des ersten Widerstands des Heizerelements, von denen beide als unabhängige Variablen in die Heizwertberechnungsformel eingesetzt sind, durch den gemessenen Wert des zweiten Widerstands des Temperaturmesselements, das mit dem gemischten Gas in Kontakt gelangt, und den korrigierten gemessenen Wert des ersten Widerstands des Heizerelements, das mit dem gemischten Gas in Kontakt gelangt, um so einen Heizwert des gemischten Gases zu berechnen.

15. Verfahren nach Anspruch 14, ferner umfassend:

(e) Erhalten eines gemessenen Werts des zweiten Widerstands des Temperaturmesselements, das mit dem Kalibriergas in Kontakt gelangt, und des gemessenen Werts des ersten Widerstands des Heizerelements, das mit dem Kalibriergas in Kontakt gelangt;
(f) Substituieren des zweiten Widerstands des Temperaturmesselements und des ersten Widerstands des Heizerelements, von denen beide als unabhängige Variablen in die Heizwertberechnungsformel eingesetzt sind, durch den gemessenen Wert des zweiten Widerstands und den gemessenen Wert des ersten Widerstands, der durch den Korrekturkoeffizienten korrigiert ist, um so einen berechneten Wert des Heizwerts des Kalibriergases zu erhalten; und
(g) Korrigieren der Heizwertberechnungsformel, so dass der berechnete Heizwert des Kalibriergases gleich dem Heizwert des Kalibriergases ist, der im Voraus erhalten wird.

16. Verfahren nach Anspruch 14 oder 15,
wobei mehrere Spannungen an das Heizerelement angelegt werden, und
die Anzahl der Spannungen größer oder gleich einer Anzahl ist, die um Eins kleiner ist als die Anzahl der mehreren Gase, die in dem gemischten Gas enthalten sind.

**Revendications**

1.  Système de mesure de résistance comprenant :

    un dispositif de stockage de formule de calcul de résistance (421) qui est arrangé pour stocker une formule de calcul de résistance, dans lequel la formule de calcul de résistance est représentée par :

    une première température détectée par un élément de mesure de température (62) entrant en contact avec un gaz de calibration, la première température étant définie comme une variable indépendante dans la formule de calcul de résistance ; and
    une première résistance d'un élément chauffant (61) entrant en contact avec le gaz de calibration, la résistance étant définie comme une variable dépendante dans la formule de calcul de résistance,

    un premier module de mesure (321) qui est arrangé pour obtenir une valeur mesurée de la première température et une valeur mesurée de la première résistance ;
    un module de calcul de coefficient de correction (322) qui est arrangé pour :

    substituer la valeur mesurée de la première température dans la première température qui est définie comme la variable indépendante dans la formule de calcul de résistance de façon à obtenir une valeur calculée de la première résistance ; et
    calculer un coefficient de correction qui représente un rapport entre la valeur mesurée de la première résistance et la valeur calculée de la première résistance ; et

    un module de correction de mesure (323) qui corrige une valeur mesurée de la première résistance, sur la base du coefficient de correction.

2.  Système de création de formule de calcul de valeur calorifique (20) comprenant :

    un deuxième module de mesure (301) qui est arrangé pour obtenir :

    une valeur mesurée d'une deuxième résistance de l'élément de mesure de température entrant en contact avec un gaz mélangé incluant une pluralité de gaz ; et
    une valeur mesurée de la première résistance de l'élément chauffant entrant en contact avec le gaz mélangé incluant la pluralité de gaz,

    le système de mesure de résistance selon la revendication 1, dans lequel le module de correction de mesure (323) est arrangé pour corriger la valeur mesurée de la première résistance de l'élément chauffant entrant en contact avec le gaz mélangé, sur la base du coefficient de correction ; et
    un module de création de formule de calcul de valeur calorifique (302) qui est arrangé pour créer une formule de calcul de valeur calorifique, sur la base de : valeurs calorifiques de la pluralité de gaz inclue dans le gaz mélangé ; la valeur mesurée de la deuxième résistance de l'élément de mesure de température entrant en contact avec le gaz mélangé ; et la valeur de mesure corrigée de la première résistance de l'élément chauffant,

    dans lequel la formule de calcul de valeur calorifique est représentée par :

    la deuxième résistance de l'élément de mesure de température qui est définie comme une variable indépendante dans la formule de calcul de valeur calorifique ;
    la première résistance de l'élément chauffant qui est définie comme une variable indépendante dans la formule de calcul de valeur calorifique ; et
    une valeur calorifique du gaz mélangé qui est définie comme une variable dépendante dans la formule de calcul de valeur calorifique.

3.  Système selon la revendication 2, comprenant en outre :

    un troisième module de mesure (325) qui est arrangé pour obtenir :

    une valeur mesurée de la deuxième résistance de l'élément de mesure de température entrant en contact avec le gaz de calibration ; et

la valeur mesurée de la première résistance de l'élément chauffant entrant en contact avec le gaz de calibration,

un module de calcul de valeur calorifique (305) qui est arrangé pour substituer la valeur mesurée de la deuxième résistance et la valeur mesurée de la première résistance qui est corrigée par le coefficient de correction, dans la deuxième résistance de l'élément de mesure de température et la première résistance de l'élément chauffant, les deux étant définies comme des variables indépendantes dans la formule de calcul de valeur calorifique, de façon à obtenir une valeur calculée d'une valeur calorifique du gaz de calibration ; et
un module de correction de formule de calcul de valeur calorifique (327) qui corrige la formule de calcul de valeur calorifique de sorte que la valeur calculée de la valeur calorifique du gaz de calibration est égale à la valeur calorifique du gaz de calibration qui est obtenue en avance.

**4.** Système selon la revendication 2 ou 3,
dans lequel une pluralité de tensions est appliquée à l'élément chauffant, et le nombre des tensions est supérieur ou égal à un nombre qui est inférieur d'un au nombre de la pluralité de gaz inclue dans la gaz mélangé.

**5.** Système selon l'une quelconque des revendications 2 à 4,
dans lequel le module de création de formule de calcul de valeur calorifique est arrangé pour créer la formule de calcul de valeur calorifique en utilisant une régression à vecteur support.

**6.** Système de mesure de valeur calorifique comprenant :

un deuxième module de mesure (331) qui est arrangé pour obtenir :

une valeur mesurée d'une deuxième résistance de l'élément de mesure de température (62) entrant en contact avec un gaz mélangé incluant une pluralité de gaz ; et
une valeur mesurée de la première résistance de l'élément chauffant entrant en contact avec le gaz mélangé ;

le système de mesure de résistance selon la revendication 1, dans lequel le module de correction de mesure (323) est arrangé pour corriger la valeur mesurée de la première résistance de l'élément chauffant entrant en contact avec le gaz mélangé, sur la base du coefficient de correction ;
un dispositif de stockage de formule de calcul de valeur calorifique (402) qui est arrangé pour stocker une formule de calcul de valeur calorifique, dans lequel la formule de calcul de valeur calorifique est représentée par :

la deuxième résistance de l'élément de mesure de température qui est définie comme une variable indépendante dans la formule de calcul de valeur calorifique ;
la première résistance de l'élément chauffant qui est définie comme une variable indépendante dans la formule calcul de valeur calorifique ; et
une valeur calorifique qui est définie comme une variable dépendante dans la formule de calcul de valeur calorifique ; et

un module de calcul de valeur calorifique (305) qui est arrangé pour substituer la valeur mesurée de la deuxième résistance de l'élément de mesure de température entrant en contact avec le gaz mélangé et la valeur de mesure corrigée de la première résistance de l'élément chauffant entrant en contact avec le gaz mélangé, dans la deuxième résistance de l'élément de mesure de température et la première résistance de l'élément chauffant, les deux étant définies comme des variables indépendantes dans la formule de calcul de valeur calorifique, de façon à calculer une valeur calorifique du gaz mélangé.

**7.** Système selon la revendication 6, comprenant en outre :

un troisième module de mesure (325) qui est arrangé pour obtenir :

une valeur mesurée de la deuxième résistance de l'élément de mesure de température entrant en contact avec le gaz de calibration ; et
la valeur mesurée de la première résistance de l'élément chauffant entrant en contact avec le gaz de calibration ; et

un module de correction de formule de calcul de valeur calorifique (327) qui est arrangé pour corriger la formule de calcul de valeur calorifique de sorte que la valeur calorifique calculée du gaz de calibration est égale à la valeur calorifique du gaz de calibration qui est obtenue en avance,

dans lequel le module de calcul de valeur calorifique est arrangé pour substituer la valeur mesurée de la deuxième résistance et la valeur mesurée de la première résistance qui est corrigée par le coefficient de correction, dans la deuxième résistance de l'élément de mesure de température et la première résistance de l'élément chauffant, les deux étant définies comme des variables indépendantes dans la formule de calcul de valeur calorifique, de façon à obtenir une valeur calculée de valeur calorifique du gaz de calibration.

8. Système selon la revendication 6 ou 7,
dans lequel une pluralité de tensions est appliquée à l'élément chauffant, et
le nombre de tensions est supérieur ou égal à un nombre qui est inférieur d'un au nombre de la pluralité de gaz inclue dans le gaz mélangé.

9. Procédé de mesure d'une résistance, la méthode comprenant :

(a) préparer une formule de calcul de résistance, dans lequel la formule de calcul de résistance est représentée par :

une première température détectée par un élément de mesure de température (62) entrant en contact avec un gaz de calibration, la première température étant définie comme une variable indépendante dans la formule de calcul de résistance ; et
une première résistance d'une élément chauffant (61) entrant en contact avec le gaz de calibration, la résistance étant définie comme une variable dépendante dans la formule de calcul de résistance,

(b) obtenir une valeur mesurée de la première température et une valeur mesurée de la première résistance ;
(c) substituer la valeur mesurée de la première température dans la première température qui est définie comme la variable indépendante dans la formule de calcul de résistance de façon à obtenir une valeur calculée de la première résistance ; et
(d) calculer un coefficient de correction qui représente a rapport entre la valeur mesurée de la première résistance et la valeur calculée de la première résistance ; et
(e) corriger une valeur mesurée de la première résistance, sur la base du coefficient de correction.

10. Procédé de création d'une formule de calcul de valeur calorifique, le procédé comprenant déterminer un coefficient de correction selon la revendication 9, et comprenant les étapes suivantes :

(a) obtenir une valeur mesurée d'une deuxième résistance d'un élément de mesure de température entrant en contact avec un gaz mélangé incluant une pluralité de gaz ;
(b) obtenir une valeur mesurée d'une première résistance d'un élément chauffant entrant en contact avec le gaz mélangé incluant la pluralité de gaz.
(c) corriger la valeur mesurée de la première résistance de l'élément chauffant entrant en contact avec le gaz mélangé, sur la base dudit coefficient de correction ; et
(d) créer une formule de calcul de valeur calorifique, basée sur : les valeurs calorifiques de la pluralité de gaz inclue dans le gaz mélangé, la valeur mesurée de la deuxième résistance de l'élément de mesure de température entrant en contact avec le gaz mélangé ; et la valeur mesurée corrigée de la première résistance de l'élément chauffant, dans lequel la formule de calcul de valeur calorifique est représentée par :

la deuxième résistance de l'élément de mesure de température qui est définie comme une variable indépendante dans la formule de calcul de valeur calorifique ;
la première résistance de l'élément chauffant qui est définie comme une variable indépendante dans la formule de calcul de valeur calorifique ; et
une valeur calorifique du gaz mélangé qui est définie comme une variable dépendante dans la formule de calcul de valeur calorifique.

11. Procédé selon la revendication 10, comprenant en outre :

(e) obtenir une valeur mesurée de la deuxième résistance de l'élément de mesure de température entrant en contact avec un gaz de calibration, et la valeur mesurée de la première résistance de l'élément chauffant entrant en contact avec le gaz de calibration,

(f) substituer la valeur mesurée de la deuxième résistance et la valeur mesurée de la première résistance qui est corrigée par le coefficient de correction, dans la deuxième résistance de l'élément de mesure de température et la première résistance de l'élément chauffant, les deux étant définies comme des variables indépendantes dans la formule de calcul de valeur calorifique, de façon à obtenir une valeur calculée d'une valeur calorifique du gaz de calibration ; et

(g) corriger la formule de calcul de valeur calorifique de sorte que la valeur calculée de la valeur calorifique du gaz de calibration est égale à la valeur calorifique du gaz de calibration qui est obtenue en avance.

**12.** Procédé selon la revendication 10 ou 11,

dans lequel une pluralité de tensions est appliquée à l'élément chauffant, et

le nombre des tensions est supérieur ou égal à un nombre qui est inférieur d'un au nombre de la pluralité de gaz inclue dans le gaz mélangé.

**13.** Procédé selon l'une quelconque des revendications 10 à 12,

dans lequel l'étape (d) comprend : créer la formule de calcul de valeur calorifique en utilisant une régression à support vecteur.

**14.** Procédé de mesure d'une valeur calorifique, le procédé comprenant :

(a) obtenir une valeur mesurée d'une deuxième résistance d'un élément de mesure de température (62) entrant en contact avec un gaz mélangé incluant une pluralité de gaz, et une valeur mesurée d'une première résistance d'un élément chauffant entrant en contact avec le gaz mélangé ;

(b) corriger la valeur mesurée de la première résistance de l'élément chauffant entrant en contact avec le gaz mélangé, sur la base d'un coefficient de correction par le procédé selon la revendication 9 ;

(c) préparer une formule de calcul de valeur calorifique, dans lequel la formule de calcul de valeur calorifique est représentée par :

la deuxième résistance de l'élément de mesure de température qui est définie comme une variable indépendante dans la formule de calcul de valeur calorifique ;

la première résistance de l'élément chauffant qui est définie comme une variable indépendante dans la formule de calcul de valeur calorifique ; et

une valeur qui est définie comme une variable dépendante dans la formule de calcul de valeur calorifique,

(d) substituer la valeur mesurée de la deuxième résistance de l'élément de mesure de température entrant en contact avec le gaz mélangé et la valeur mesurée corrigée de la première résistance de l'élément chauffant entrant en contact avec le gaz mélangé, dans la deuxième résistance de l'élément de mesure de température et la première résistance de l'élément chauffant, les deux étant définies comme des variables indépendantes dans la formule de calcul de valeur calorifique, de façon à calculer une valeur calorifique du gaz mélangé.

**15.** Procédé selon la revendication 14, comprenant en outre :

(e) obtenir une valeur mesurée de la deuxième résistance de l'élément de mesure de température entrant en contact avec un gaz de calibration, et la valeur mesurée de la première résistance de l'élément chauffant entrant en contact avec le gaz de calibration ;

(f) substituer la valeur mesurée de la deuxième résistance et la valeur mesurée de la première résistance qui est corrigée par le coefficient de correction, dans la deuxième résistance de l'élément de mesure de température et la première résistance de l'élément chauffant, les deux étant définies comme des variables indépendantes dans la formule de calcul de valeur calorifique, de façon à obtenir une valeur calculée de la valeur calorifique du gaz de calibration ; et

(g) corriger la formule de calcul de valeur calorifique de sorte que la valeur calorifique calculée du gaz de calibration est égale à la valeur calorifique du gaz de calibration qui est obtenue en avance.

**16.** Procédé selon la revendication 14 à 15,

dans lequel une pluralité de tensions est appliquée à l'élément chauffant, et

le nombre des tensions est supérieur ou égal à un nombre qui est inférieur d'un au nombre de la pluralité de gaz inclue dans le gaz mélangé.

FIG. 1

*FIG. 2*

*FIG. 3*

*FIG. 4*

*FIG. 5*

Y-axis: RADIATION COEFFICIENT (W/℃)

X-axis: TEMPERATURE OF HEAT GENERATING RESISTOR (℃)

Legend:
- ◆ METHANE
- ✳ PROPANE
- ✕ NITROGEN
- ▩ CARBON DIOXIDE

## FIG. 6

*FIG. 7*

*FIG. 8*

```
                    ┌──────────────┐
                    │    START     │
                    └──────┬───────┘
                           │
         ┌─────────────────┼───────────────┐
         │                 ▼               │
         │    ┌────────────────────────┐   │
         │    │ PREPARE SAMPLE MIXED GAS│──── S100
         │    └────────────┬───────────┘   │
         │  ┌──────────────┼──────────┐    │
         │  │              ▼          │    │
         │  │   ┌────────────────────┐│    │
         │  │   │ OUTPUT ELECTRIC SIGNAL│── S101
         │  │   └─────────┬──────────┘│    │
         │  │             ▼           │    │
         │  │          ╱────────╲     │    │
         │  │  NO    ╱ IS SWITCHING╲──── S102
         │  └───────  OF TEMPERATURE │    │
         │          ╲  COMPLETED? ╱       │
         │            ╲────┬───╱           │
         │                 │ YES           │
         │                 ▼               │
         │              ╱────────╲         │
         │     NO     ╱ IS SWITCHING╲───── S103
         └──────────── OF SAMPLE MIXED GAS │
                      ╲  COMPLETED? ╱
                        ╲────┬───╱
                             │ YES
                             ▼
                    ┌──────────────┐
                    │ COLLECT DATA │──── S104
                    └──────┬───────┘
                           ▼
             ┌──────────────────────────┐
             │ MULTIPLE REGRESSION ANALYSIS│── S105
             └────────────┬─────────────┘
                           ▼
                    ┌──────────────┐
                    │ REGISTRATION │──── S106
                    └──────┬───────┘
                           ▼
                    ┌──────────────┐
                    │     END      │
                    └──────────────┘
```

## FIG. 9

20

| 421 | 401 |
|---|---|
| RESISTANCE CALCULA-TION FORMULA STORAGE DEVICE | ELECTRIC SIGNAL STORAGE DEVICE |

| 422 | 402 |
|---|---|
| CORRECTION COEFFI-CIENT STORAGE DEVICE | CALORIFIC VALUE CALCULATION FORMULA STORAGE DEVICE |

300

301     CPU

312

INPUT DEVICE

| MEASUREMENT MODULE FOR COEFFICIENT CALCULATION | 321 | MEASUREMENT MODULE FOR FORMULA CRE-ATION |
|---|---|---|

| CORRECTION COEFFI-CIENT CALCULATION MODULE | 322 | CALORIFIC VALUE CALCULATION FORMULA CREATION MODULE |
|---|---|---|

302

OUTPUT DEVICE

313

MEASUREMENT CORRECTION MODULE — 323

RESISTANCE CALCU-LATION FORMULA CREATION MODULE — 324

303 — DRIVING CIRCUIT

18     101

102     8     103

*FIG. 10*

*FIG. 11*

ELECTRIC SIGNAL STORAGE DEVICE —401

CALORIFIC VALUE CALCULATION FORMULA STORAGE DEVICE —402

CALORIFIC VALUE STORAGE DEVICE —403

—300

331

MEASUREMENT MODULE FOR CALORIFIC VALUE CALCULATION

301    CPU

MEASUREMENT MODULE FOR FORMULA CREATION

312

INPUT DEVICE

302

CALORIFIC VALUE CALCULATION FORMULA CREATION MODULE

305

CALORIFIC VALUE CALCULATION MODULE

OUTPUT DEVICE

313

303 — DRIVING CIRCUIT

18    101

102    8    103

—21

*FIG. 12*

```
            ┌─────────────┐
            │    START     │
            └──────┬───────┘
                   │
                   ▼
    ┌──────────────────────────────┐
    │ PREPARE MEASUREMENT TARGET MIXED │── S200
    │             GAS               │
    └──────────────┬───────────────┘
                   │
      ┌────────────│
      │            ▼
      │   ┌──────────────────────┐
      │   │ OUTPUT ELECTRIC SIGNAL │── S201
      │   └──────────┬───────────┘
      │              │              S202
      │              ▼
      │         ╱─────────────╲
      │ NO     ╱  IS SWITCHING  ╲
      └───────┤  OF TEMPERATURE  │
              ╲   COMPLETED?    ╱
               ╲───────────────╱
                   │
                  YES
                   │
                   ▼
    ┌──────────────────────────────┐
    │       READ OUT FORMULA        │── S203
    └──────────────┬───────────────┘
                   │
                   ▼
    ┌──────────────────────────────┐
    │    CALCULATE CALORIFIC VALUE   │── S204
    └──────────────┬───────────────┘
                   │
                   ▼
            ┌─────────────┐
            │     END      │
            └─────────────┘
```

## FIG. 13

## FIG. 14

FIG. 15

**EP 2 369 338 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2004514138 T
- EP 1947540 A

- JP 5141999 A

**Non-patent literature cited in the description**

- **A.J. SMOLA ; B. SCHOLKOPF.** A Tutorial on Support Vector Regression. *NeuroCOLT Technical Report (NC-TR-98-030),* 1998